# EUROPÄISCHE PATENTANMELDUNG

(11) **EP 1 980 567 A1**
(43) Veröffentlichungstag der Anmeldung: **15.10.2008**
(21) Anmeldenummer: 08012073.6
(22) Anmeldetag: 24.03.2006
(51) Int. Cl.: C07H 15/08, C07H 15/04, B01F 17/00, C11D 1/66, C11D 3/22, C11D 1/83, A61K 8/60, A61Q 19/10, A61Q 9/02, A61Q 1/14, A61Q 11/00, A61Q 5/02, C11D 1/14

(54) **Neue Tenside und neue Emulgatoren auf der Basis von Disacchariden**

(30) Priorität: 24.03.2005 DE 102005014424
(62) Teilanmeldung aus: 06723701.6
(71) Anmelder: Beiersdorf AG, 20253 Hamburg (DE)
(72) Erfinder: Schreiber, Jörg, Dr., 22087 Hamburg (DE); Milkereit, Götz, 22179 Hamburg (DE); Gerber, Sven, 22159 Hamburg (DE); Vill, Volkmar, Prof. Dr., 20146 Hamburg (DE)

(57) **Zusammenfassung**

Kosmetische Zubereitung enthaltend eine Verbindung gemäß der Formel

A-(B)ₓ-C

wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 8 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat.

## Beschreibung

Die vorliegende Erfindung betrifft neue Tenside und Emulgatoren auf der Basis von Disacchariden. Diese zeichnen sich insbesondere durch ein besonderes Schaumverhalten und eine besondere Milde bei der Anwendung auf der Haut aus. Darüber hinaus eignet sich eine Untergruppe als Gelbildner.
Der Stand der Technik kennt *ionische Tenside* wie Laurylethersulfat, Laurylsulfat. Bei diesen handelt es sich um die Standardtenside, die in Körperpflegeprodukten eingesetzt werden. Sie sind gekennzeichnet durch hohe Einsatzkonzentration (von 8-20 Gew.%) und sind nur begrenzt hautfreundlich, daher werden ihre Eigenschaften durch Abmischung, beispielsweise mit Cotensiden wie Cocamidopropylbetain und Cocoamphoacetat optimiert. Zur Erzielung einer auf der Hand schäumenden Zubereitung werden üblicherweise 10-15 Gew.% an Lauryethersulfat eingesetzt.
Ferner ist Laurylethersulfat bzw. Laurylsulfat ein Aniontensid. Die Kombination mit kationischen Tensiden ist wegen der Niederschlagsbildung in wässrigen Systemen häufig ein großes Problem (Entgegengesetzte Ladung von Anion- und Kationtensid). Ferner sind kationische Polymere für entsprechende Aniontenside aus dem gleichen Grund häufig problematisch.

Zur Verbesserung der Hautverträglichkeit von Laurylethersulfat-Formulierungen werden auch *nichtionische Tenside* wie Alkylpolyglycoside (APG) eingesetzt. APG ist das Standardtensid für Waschmittel und kosmetische Zubereitungen. Die Zusammensetzung dieser Tensidmischung ist allerdings extrem inhomogen. Seine Eigenschaften können daher nicht einer einzelnen Verbindung zugeschrieben werden. Ein bekanntes APG (Plantaren 2000 UP, Fa. Cognis) enthält beispielsweise um 1 Gew.% C₆-Glucoside, 33-40 Gew.% C₈-Glucoside, 21-28 Gew.% C₁₀-Glucoside, 27-32Gew.% C₁₂-Glucoside, 9-12 C₁₄-Glucoside, um 1 C₁₆-Glucoside sowie höhere Polymere und freien Fettalkohol. Ferner ist noch zu beachten, daß es sich dabei noch um ein Gemisch der α- und β-Anomeren der Glucoside handelt. Die Herstellung der hydrophilen Komponente des APGs erfolgt a) durch Hydrolyse von Stärke oder Dextrosesyrup oder b) aus wasserfreier Glucose (bzw. Glucosemonohydrat). Diese hydrophile Komponente wird beim Stärkehydrolyse-Weg mit Butanol in das entsprechende Glycosid überführt und anschließend mit dem langkettigen Fettalkohol umacetalisiert. Der Syntheseweg über die wasserfreie Glucose nutzt die direkte Acetalisierung mit dem langkettigen Fettalkohol in das entsprechende Glycosid. Tenside auf Disaccharid-Basis sind bislang nur von untergeordneter Bedeutung in der Kosmetik, als Waschmittel oder im Lebensmittelbereich. Alkylglycoside mit Disaccharid Kohlenhydratkopfgruppen sind ebenfalls in der Literatur beschrieben worden, so findet man z.B. Maltoside, Lactoside oder Melibioside mit Alkylkettenlängen von 10-20 Kohlenstoffatomen. Ein Nachteil dieser Verbindungen ist ihre schlechte Wasserlöslichkeit bedingt durch eine hohe Krafft-Temperatur.
Sie eignen sich daher nicht als Tenside.
Ein weiterer Nachteil der Alkyl-Glycoside wie APGs ist Ihre geringe Schaumeigenschaft in Wasser, der weiterhin einen Einsatz von Laurylethersulfat, Laurylsulfat oder anderen Cotensiden erfordert. Weiterhin sind diese Verbindungen nicht in der Lage, Gele in wässriger Lösung zu bilden, wenn die Einsatzkonzentration in Wasser kleiner als 15 Gew.% ist.

Überraschend und für den Fachmann nicht vorhersehbar werden die Nachteile des Standes der Technik durch Verbindungen gemäß der Formel A-(B)ₓ-C wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 10 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat, beseitigt.

Solche Verbindungen weisen folgende Vorteile auf:
sie sind sehr gute Schaumbildner bei Raumtemperatur und auch bei höherer Temperatur, zum Beispiel bei 50°C,
sie bilden sehr stabile Schäume,
sie bilden sehr viel Schaum bei geringen Einsatzkonzentrationen,
sie erlauben eine sehr gute Schaumbildung auch für durch den Herstellungsprozess bedingte Gemische,
sie eine einfache Herstellung,
bestimmte Verbindungen erlauben Gelbildung,
sie sind mit Cotensiden mischbar,
sie sind gut bis sehr gut verträglich.

Eine bekannte Methode die Löslichkeit von Amphiphilen zu verbessern ist die Ethoxylierung von Hydroxygruppen. So beschreibt z.B. US3640998 die Herstellung von ethoxylierten Alkylpolyglucosiden, bei denen alle freien Hydroxygruppen ethoxyliert werden. Eine gezielte Ethoxylierung in Form von ethoxylierten Alkyl-Disacchariden wurde nur selten offenbart.

Der Einsatz von Ethylenglycol als Spacer zwischen der Alkylkette eines Fettalkohols und der Kohlenhydratkopfgruppe am anomeren Zentrum ist bislang nur für wenige Verbindungen bekannt und die Eigenschaften wurden nur im Hinblick auf eine mögliche medizinische Anwendung hin untersucht (Bendas, G., Wilhelm, F., Richter, W., Nuhn, P., Eur. J. Pharm. Sci., 4, 1996, 211ff.; Nuhn, P., Pfaff, T., Bendas, G., Wilhelm, F., Chatterjee, S.K., Arch. Pharm., 327, 1994, 429ff.). Die Spacer dienen in diesem Fall zur Vergrößerung des Abstandes der Kohlenhydratkopfgruppe zur Oberfläche des Liposoms. Ferner werden hier Phospholipide mit den substituierten Zuckern zur Liposomenbildung eingesetzt. Die Vorteile einer gezielten Ethoxylierung in Form von ethoxylierten Alkyl-Disacchariden auf Basis von Lactose, Maltose, Mellibiose ohne Phospholipide ist nichts bekannt. Über Schaumbildung, Gelbildung oder ihre Eignung als Tensid ist nichts bekannt.

In der vorliegenden Erfindung wurden nun diverse Derivate mit der Struktur Disaccharid-Spacer-Alkylkette untersucht:

A-B-C

mit
A = Kohlenhydratkopfgruppe
B = Spacer
C = hydrophobe Kette

Es wurde gefunden, daß durch den Einsatz eines Spacers die Löslichkeit der Verbindungen in Wasser entschieden verbessert werden konnte. Auch allein der Einsatz der anomeren Mischungen, die man durch den Herstellungsprozeß bedingt oder durch Abmischung der Anomere erhält, führt zu einer verbesserten Löslichkeit. Darüberhinaus wurde gefunden, daß der Einsatz eines Glycol-Spacers die Gelbildung von Lauryl Lactosiden erlaubt. Das Schaumverhalten der Verbindungen wird ebenfalls verbessert gegenüber einer Verbindung ohne den Spacer. Ferner läßt sich durch Abmischung mit Cotensiden ein feiner Schaum erzielen. Die Verbindungen sind im Bezug auf ihre Hautverträglichkeit besser verträglich als die Standardtenside Laurylsulfat und Laurylethersulfat. Ferner benötigt man zur Erzielung eines Schaums eine geringere Einsatzkonzentration (in Abhängigkeit vom gewählten Disaccharid-Alkylglycosid etwa 1-5 Gew.%).

Als besonders vorteilhafte Ausführungsformen der Erfindung haben sich die Gegenstände der übrigen Patentansprüche herausgestellt.
Als Einheit A eignen sich glycosidisch verknüpfte Disaccharide und z.T. glycosidisch verknüpfte Trisaccharide.
Geeignet sind auch reine Anomere und Gemische der Anomere, durch physikalische Abmischung oder bedingt durch den Herstellungsprozeß, sowie Mischungen der Verbindungen untereinander, also Lactoside, Melibioside, Maltoside, Cellobiose die als jeweils reines Alpha oder Beta-Anomer eingesetzt werden bzw auch das Anomerengemisch,
Gemische von Lactosiden mit Melibiosiden, Gemische von Lactosiden mit Maltosiden und/oder Gemische von Meliosiden und Maltosiden, jeweils rein oder als Anomerengemische.

Laktose kann von der Firma Sigma, Fucka, Biolac (Arla Food-Konzern, 31097 Harbansen) bezogen werden. Ferner kann auch neben wasserfreier Laktose das Monolydrat von Laktose (Variolac 99 edible, Biolac) eingesetzt werden.

Besonders für den Einsatz als Tenside oder Emulgatoren geeignete Disaccharid-Einheiten sind Lactose, Melibiose und Maltose sowie Cellobiose:

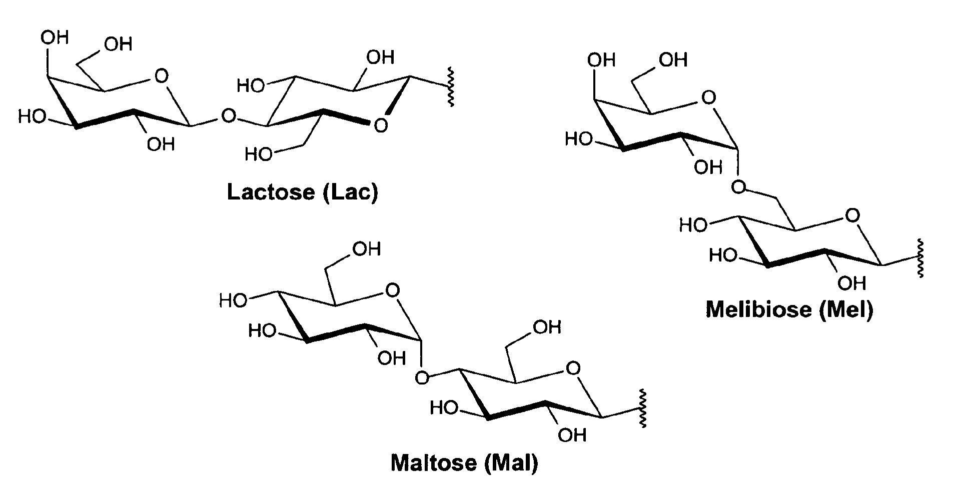

Erfindungsgemäß können sowohl deren Monohydrate, als auch die wasserfreien Verbindungen eingesetzt werden, besonders bevorzugt die Monohydrate.
Als Einheit B (Spacer) eignen sich u.a. Ethylenglycol (EG), Diethylenglycol, Triethylenglycol, Tetraethylenglycol, Propylenglycol (PG), Dipropylenglycol, Glycerin, Butylenglycol, Pentaerythrit. Besonders geeignete Einheiten B sind die folgenden Strukturen: 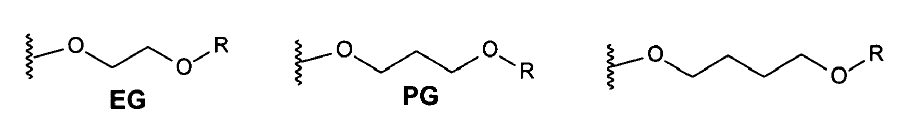 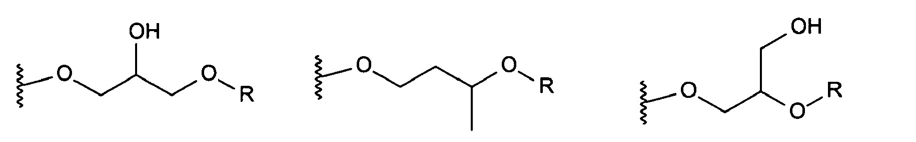 Ganz besonders bevorzugt sind Ethylenglycol, Diethylenglycol, Triethylenglycol, Tetraethylenglycol, Propan-1,2-diol, Propan-1,3-diol, Dipropylenglycol, Glycerin, Butandiol, Pentaerythrit.

Besonders geeignete Einheiten C für den Einsatz als Tensid sind allgemein Alkylketten mit 8-14 C-Atomen, die linear, gesättigt, verzweigt oder ungesättigt sein können. Besonders bevorzugt für Tenside sind lineare und gesättigte Alkylketten mit 8-12 C-Atomen.

Besonders bevorzugt als Gelbildner sind als Einheit C lineare, gesättigte Alkylketten mit 12 C-Atomen und besonders bevorzugt solche mit einer Einheit B die im Mittel eine in Form einer einzigen EO-Einheit aufweist. Als gelbildende erfindungsgemäße Produkte können auch Produkte aus Lactose und den Fettalkohol-Narrow-Range Ethoxylaten wie Berol 260 oder Berol 266 (Firma Akzo) besonders bevorzugt verwendet werden.
Bevorzugt für den Einsatz als Emulgatoren sind Verbindungen mit Kettenlängen von 12-22 C-Atomen. Besonders bevorzugt sind dann C16 bis C18 Alkylketten.
Dabei ist es von Vorteil, wenn das Verhältnis zwischen Spacer (B) und Kette (C) konstant gehalten wird, in dem Sinne, daß die Kettenlänge mit der Spacerlänge wächst.

Besonders bevorzugte Verbindungen sind insbesondere als schäumende Verbindungen:

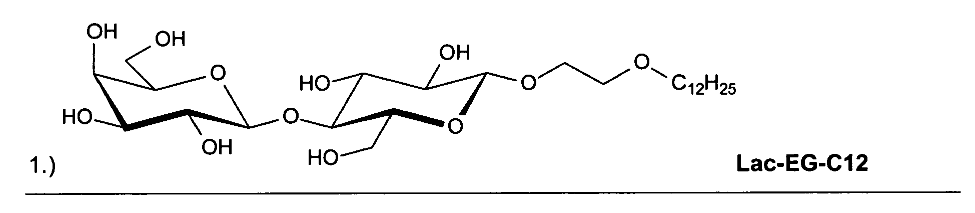

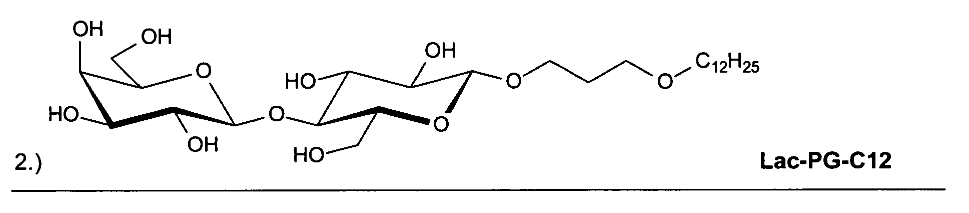

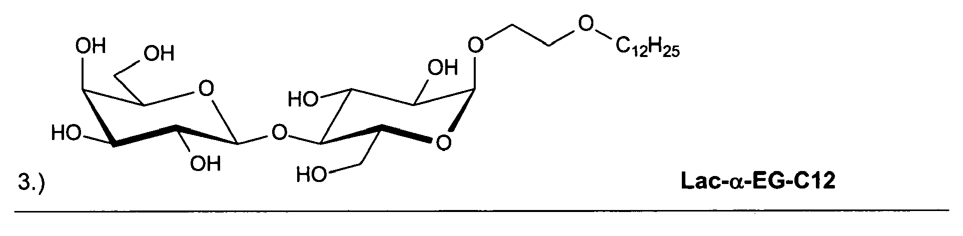

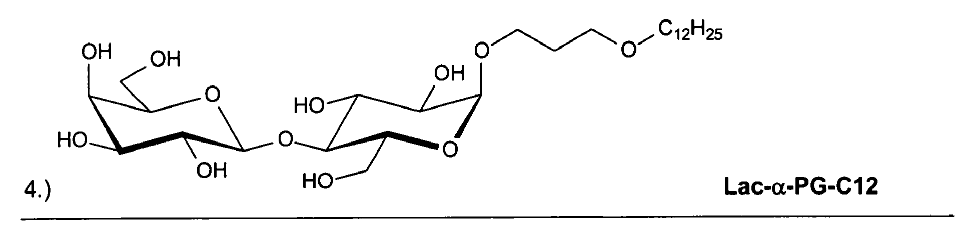

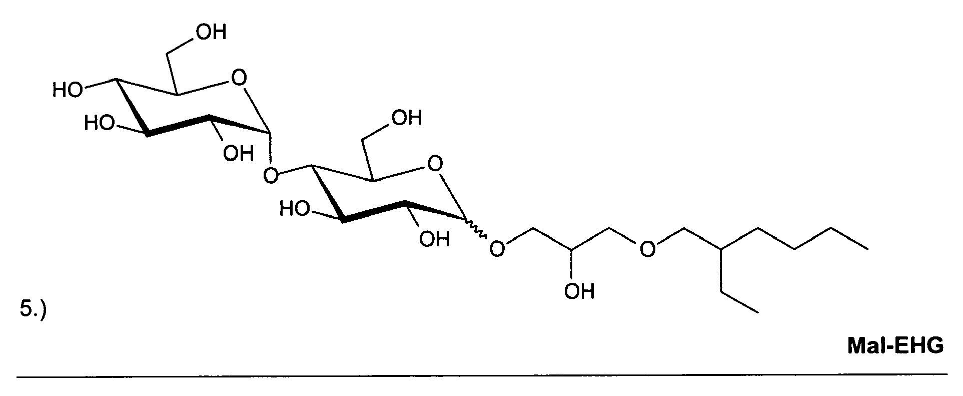

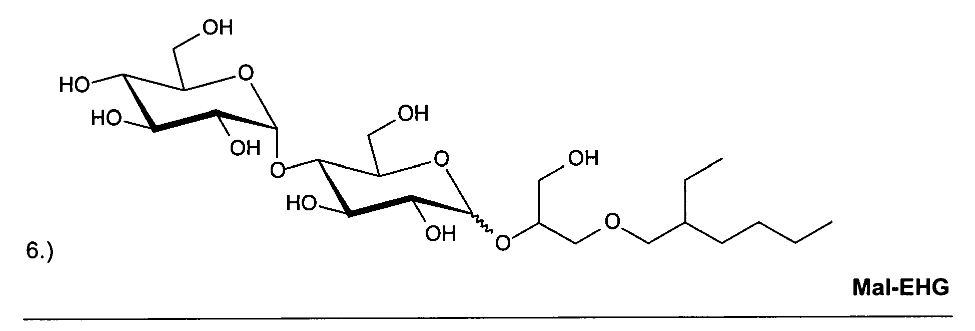

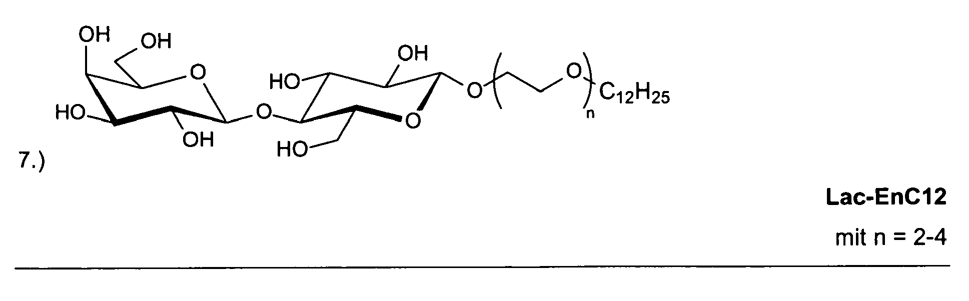

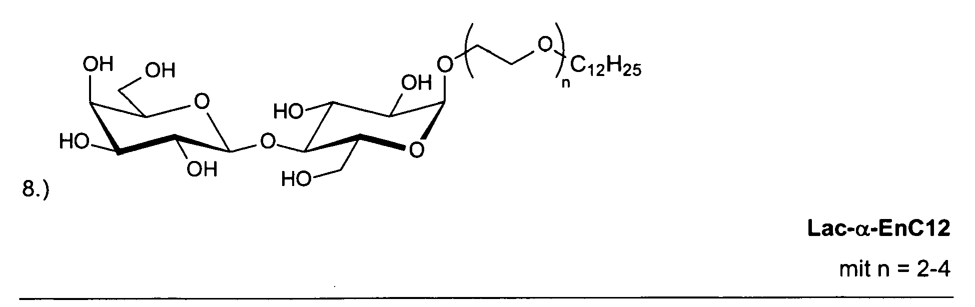

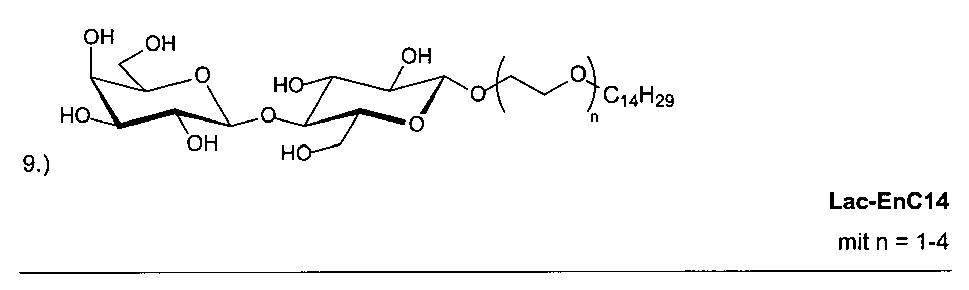

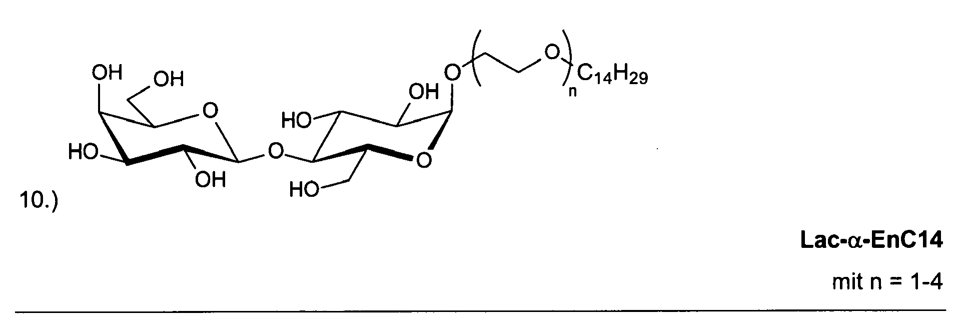

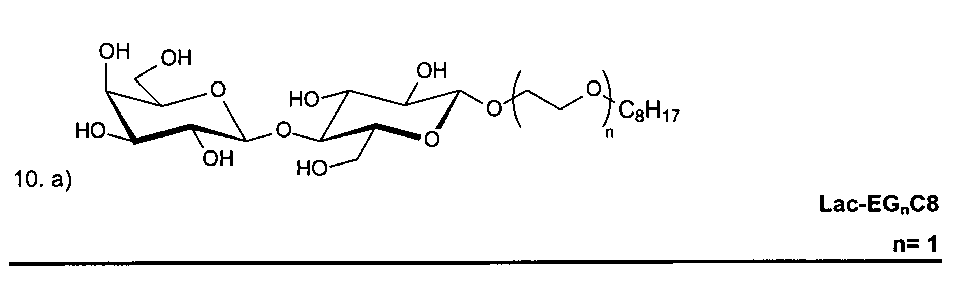

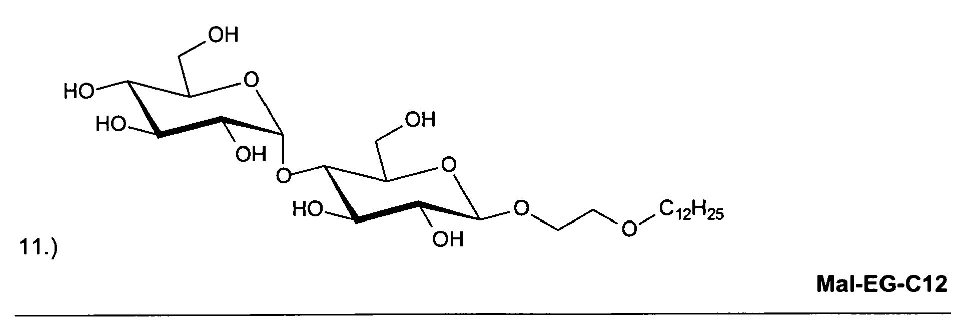

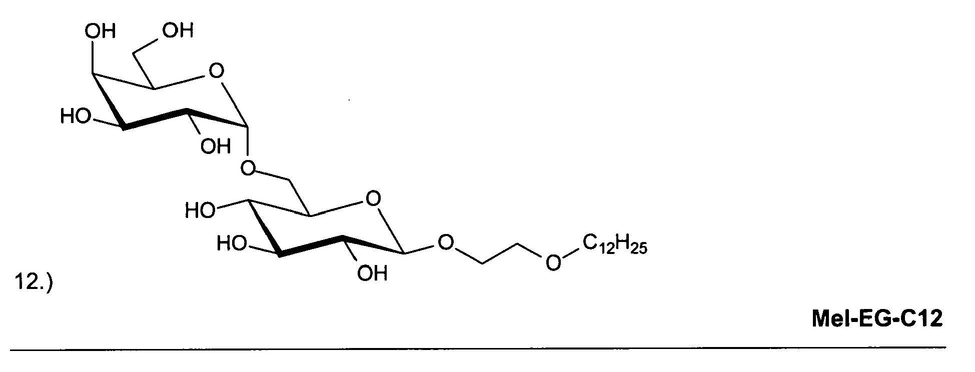

Günstig sind auch Lactoside technischer Fettalkoholethoxylate, insbesondere die folgenden Verbindungen 13 bis 16: 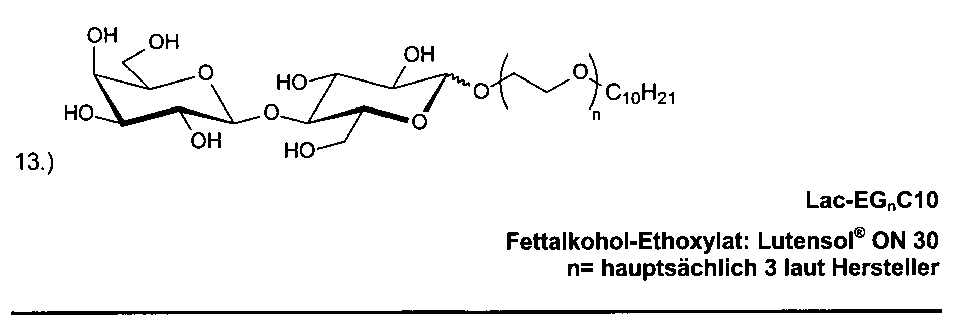 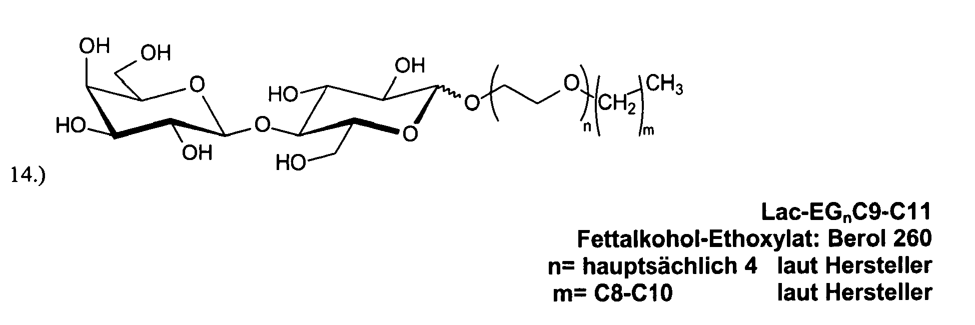 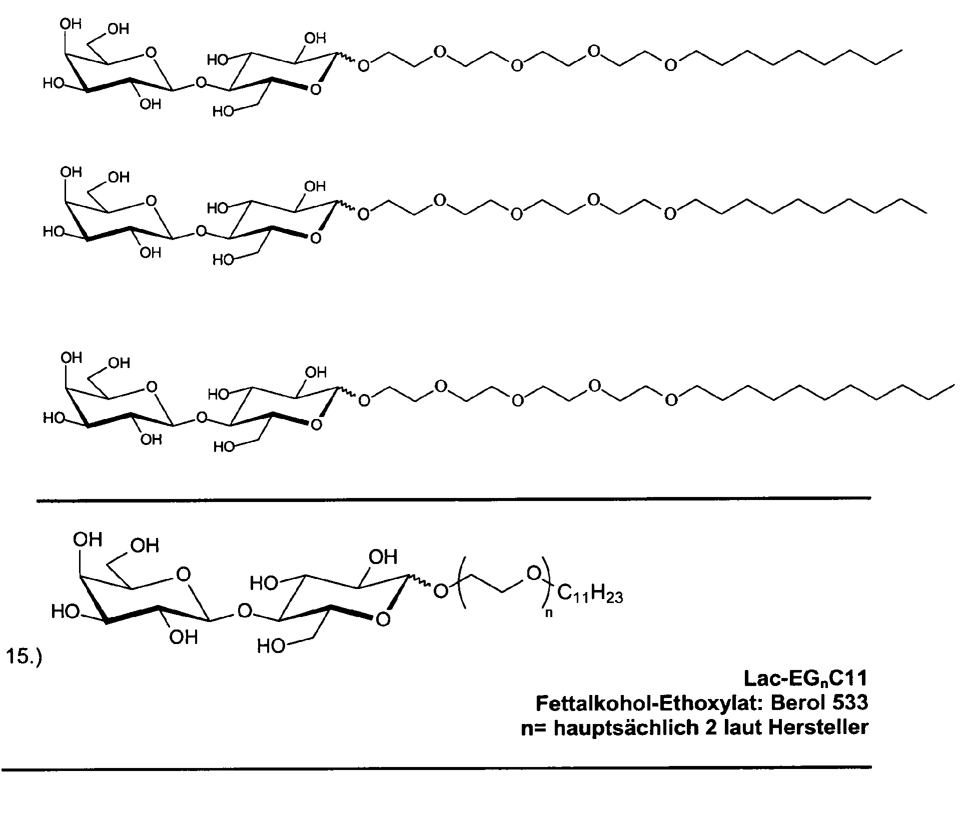 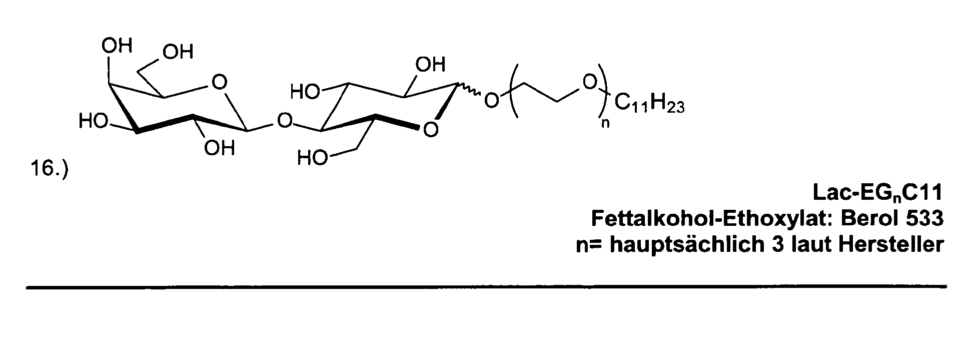
zu 1.) (2'-Dodecyloxyethyl) 4-O-(β-D-galactopyranosyl)-β-D-glucopyranosid
   (**Lac-EG-C12**),
zu 2.) (2'-Dodecyloxypropyl) 4-0-(β-D-galactopyranosyl)-β-D-glucopyranosid
   (**Lac-PG-C12**),
zu 3.) (2'-Dodecyloxyethyl) 4-*O*-(β-D-galactopyranosyl)-α-D-glucopyranosid
   **(Lac-α-EG-C12),**
zu 4.) (2'-Dodecyloxypropyl) 4-*O*-(β-D-galactopyranosyl)-α-D-glucopyranosid
   **(Lac-α-PG-C12).**
zu 5.) 1-*O*-(2'-Ethylhexyoxy)-3-*O*-(4"-*O*-α-D-glucopyranosyl)-D-glucopyranosyl)-glycerin
   (**Mal-EHG**)
zu 6.) 1-*O*-(2'-Ethylhexyoxy)-2-*O*-(4"-*O*-α-D-glucopyranosyl)-D-glucopyranosyl)-glycerin
   (**Mal-EHG**)
zu 7.) (2'-(2"-Dodecyloxyethyloxy)-ethyl) 4-*O*-(β-D-galactopyranosyl)-β-D-glucopyranosid
   (**Lac-EG2C12**)
   (2'-(2"-(2"'-Dodecyloxyethyloxy)-ethyloxy)-ethyl) 4-*O*-(β-D-galactopyranosyl)-β-D-glucopyranosid **(Lac-EG3C12)**
   (2'-(2"-(2"'-(2""-Dodecyloxyethyloxy)-ethyloxy)-ethyloxy)-ethyl) 4-*O*-(β-D-galactopyranosyl)-β-D-glucopyranosid **(Lac-EG4C12)**
zu 8.) (2'-(2"-Dodecyloxyethyloxy)-ethyl) 4-*O*-(β-D-galactopyranosyl)-α-D-glucopyranosid
   **(Lac-α-EG2C12)**
   (2'-(2"-(2"'-Dodecyloxyethyloxy)-ethyloxy)-ethyl) 4-*O*-(β-D-galactopyranosyl)-α-D-glucopyranosid **(Lac-α-EG3C12)**
   (2'-(2"-(2"'-(2""-Dodecyloxyethyloxy)-ethyloxy)-ethyloxy)-ethyl) 4-*O*-(β-D-galactopyranosyl)-α-D-glucopyranosid **(Lac-α-EG4C12)**
zu 9.) (2'-Tetradecyloxyethyl) 4-O-(β-D-galactopyranosyl)-β-D-glucopyranosid **(Lac-EG1C14)**
   (2'-(2"-Tetradecyloxyethyloxy)-ethyl) 4-O-(β-D-galactopyranosyl)-β-D-glucopyranosid
   (**Lac-EG2C14**)
   (2'-(2"-(2"'-Tetradecyloxyethyloxy)-ethyloxy)-ethyl)4-*O*-(β-D-galactopyranosyl)-β-D-glucopyranosid **(Lac-EG3C14)**
   (2'-(2"-(2"'-(2""-Tetradecyloxyethyloxy)-ethyloxy)-ethyloxy)-ethyl) 4-*O*-(β-D-galactopyranosyl)-β-D-glucopyranosid **(Lac-EG4C14)**
zu 10.) (2'-Tetradecyloxyethyl) 4-*O*-(β-D-galactopyranosyl)-α-D-glucopyranosid
   **(Lac-α-EG1C14)**
   (2'-(2"-Tetradecyloxyethyloxy)-ethyl) 4-*O*-(β-D-galactopyranosyl)-α-D-glucopyranosid
   **(Lac-α-EG2C14)**
   (2'-(2"-(2"'-Tetradecyloxyethyloxy)-ethyloxy)-ethyl) 4-*O*-(β-D-galactopyranosyl)-α-D-glucopyranosid
   **(Lac-α-EG3C14)**
   (2'-(2"-(2"'-(2""-Tetradecyloxyethyloxy)-ethyloxy)-ethyloxy)-ethyl) 4-*O*-(β-D-galactopyranosyl)-α-D-glucopyranosid
   **(Lac-α-EG4C14)**
zu 10a)
   (2'-(Octyloxyethyl)-4-O-(β-D-galactopyranosyl)-β-D-glucopyranosid
   (**Lac-EGC8**)
zu 11.) (2'-Dodecyloxyethyl) 4-*O*-(α-D-glucopyranosyl)-β-D-glucopyranosid
   (**Mal-EG-C12**)
zu 12.)(2'-Dodecyloxyethyl) 6-*O*-(α-D-galactopyranosyl)-β-D-glucopyranosid
   (**Mel-EG-C12**)
Zu 13.)
   **Laktosid: Lac-EG₃C10 und Lac-α-EG₃C10**
(2-[2-[2-(Decyloxy) ethoxy] ethoxy] ethyl)-4-O-(β-D-galactopyranosyl)-β-D-glucopyranosid
   **(Lac-EG₃C10)**
(2-[2-[2-(Decyloxy) ethoxy] ethoxy] ethyl)-4-*O*-(β-D-galactopyranosyl)-α-D-glucopyranosid C₂₈H₅₄O₁₄
   **(Lac-α-EG₃C10)**
Zu 14.)
   **Laktosid: Lac-EG₄C9 und Lac-α-EG₄C9**
   **Laktosid: Lac-EG₄C10 und Lac-α-EG₄C10**
   **Laktosid: Lac-EG₄C11 und Lac-α-EG₄C11**
(2-[2-[2-[2-(Nonyloxy) ethoxy] ethoxy] ethoxy)] ethyl)-4-*O*-(β-D-galactopyranosyl)-β-D-glucopyranosid
   **(Lac-EG₄C9)**
(2-[2-[2-[2-(Nonyloxy) ethoxy] ethoxy] ethoxy)] ethyl)-4-*O*-(β-D-galactopyranosyl)-α-D-glucopyranosid
   **(Lac-α-EG₄C9)**
(2-[2-[2-[2-(Decyloxy)ethoxy] ethoxy] ethoxy)] ethyl)-4-*O*-(β-D-galactopyranosyl)-β-D-glucopyranosid
   **Lac-EG₄C10**
(2-[2-[2-[2-(Decyloxy) ethoxy] ethoxy] ethoxy)] ethyl)-4-*O*-(β-D-galactopyranosyl)-α-D-glucopyranosid
   **(Lac-α-EG₄C10)**
(2-[2-[2-[2-(Undecyloxy) ethoxy] ethoxy] ethoxy)] ethyl)-4-*O*-(β-D-galactopyranosyl)-β-D-glucopyranosid
   **Lac-EG₄C11**
(2-[2-[2-[2-(Undecyloxy) ethoxy] ethoxy] ethoxy)] ethyl)-4-*O*-(β-D-galactopyranosyl)-α-D-glucopyranosid
   **(Lac-α-EG₄C11)**
Zu 15.)
   **Laktosid: Lac-EG₂-C11 und Lac-α-EG₂-C11**
(2-[2-(Undecyloxy) ethoxy] ethyl)-4-*O*-(β-D-galactopyranosyl)-β-D-glucopyranosid
   **(Lac-EG₂C11**)
(2-[2-(Undecyloxy) ethoxy] ethyl)-4-*O*-(β-D-galactopyranosyl)-α-D-glucopyranosid
   **(Lac-α-EG₂C11)**
Zu 16.)
   **Laktosid: Lac-EG₃-C11 und Lac-α-EG₃-C11**
   (2-[2-[2-(Undecylyoxy) ethoxy] ethoxy] ethyl)-4-O-(β-D-galactopyranosyl)-β-D-glucopyranosid
   **(Lac-EG₃C11**)
   (2-[2-[2-(Undecyloxy) ethoxy] ethoxy] ethyl)-4-*O*-(β-D-galactopyranosyl)-α-D-glucopyranosid
   **(Lac-α-EG₃C11)**

### Laktoside, die Gele bilden:

Besonders als Gelbildner geeignete Einheiten sind β-1,4-verknüpfte Saccharide wie z.B. Lactose und Cellobiose. Das Tensid bildet hier ein Gel und schäumt zusätzlich.

Als besonders geeignete Gelbildner haben sich die β-Anomeren von **Lac-EG-C12** bzw. **Lac-PG-C12** erwiesen.

Ferner eignen sich Laktoside, die technische Narrow-Range -Ethoxylate wie Berol® 260 (C9-C11 EGn; n= 4 laut Akzo) enthalten.

### Schaumbildner:

Als alleinige Verbindungen zeichnen sich besonders die Verbindungen **Lac-EG-C12, Mal-EG-C12** und **Mel-EG-C12** als gute Schaumbildner aus.
Des Weiteren zeigt die Mischung aus **Lac-PG-C12** mit **Mal-EHG** ein sehr gutes Schaumverhalten.

Ferner sind auch die Mischungen die aus der Umsetzung von Lactose beziehungsweise **Laktose-Monohydrat** mit kurzkettigen, ethoxylierten Fettalkoholen' wie Laureth-2 und Laureth-3 (**Lac-Laureth-2** bzw. **Lac-Laureth-3** bzw **Lac-EG2C12** und **Lac-EG3C12**) hergestellt wurden, besonders gute Schaumbildner.

Kurzkettige Fettalkohol-Ethoxylate wie Laureth-2 bzw. Laureth-3 sind von der Firma Sasol erhältlich (Marlipal) bzw. von Akzo Nobel (Berol 260, 266, 533, 532, 840), (Ethylan 1005) und von BASF (Lutensol ON 30). Lutensol ON 30 stellt Decylalkohol mit 3 EO-Einheiten dar, die in einer technischen Mischung vorliegen. Daher liegen darin auch Moleküle mit höheren und niedrigeren Zahlen an EO-Einheiten vor. Der Fettalkohol kann auch teilweise oder ganz als iso-Fettalkohol vorliegen. Das gleiche gilt für alle technischen EO-Fettalkohole-Addukte. Berol 260, 266, 840 und Ethylan 1005 gehören zu den "narrow range"- Fettalkoholethoxylaten, diese zeichnen sich durch eine besonders enge Verteilung der Ethoxylierung aus. Beispielsweise ist das Berol 260 im Mittel mit vier Ethylenoxyeinheiten verknüpft, die Verteilungkurve ist schmaler im Vergleich zu Standard-Ethoxylaten. Dennoch können im Berol 260 Verbindungen mit 1 bis 16 Ethylenoxyeinheiten enthalten sein. Näheres findet sich im Produktdatenblatt "Berol 260, 266, 840, Ethylan 1005" des Herstellers Akzo Nobel vom September 2005.

### Zu den von Firmen wie Sasol erhältlichen Fettalkohol-Ethoxylaten:

Der Fachmann weiß, dass es sich hier nicht um reines Laureth-2 oder Laureth-3 handelt, vielmehr liegt der ethoxylierte Laurylalkohol mit 1-5 oder mehr EO-Einheiten vor.
Entsprechend mit kommerziell erhältlichem Laureth-x (x= Ethoxylierungsgrad) umgesetztes Disaccharid (z.B. Lactose) wird daher genau genommen durch die vorab beschriebenen Strukturen nicht exakt wieder gegeben.
Ferner ist kommerziell erhältliches Laureth-x (x= Ethoxylierungsgrad) nicht rein bezogen auf den Fettalkohol und kann daher sowohl längerkettige, als auch kürzerkettige Fettalkohole als Laurylalkohol enthalten.
Dementsprechend ist die Bezeichnung für das Lactosid **"Lac-Laureth-2"** bzw. **"Lac-Laureth-3"** als Umsetzungsprodukt von technischem Laureth-2 bzw. Laureth-3 mit Lactose so zu verstehen, dass auch kürzer oder längerkettige Fettalkohole ethoxyliert hier vorliegen können.

Ferner können auch reine Fettalkoholethoxylate für die Umsetzung mit dem jeweiligen Disaccharid eingesetzt werden.

Ferner konnte gezeigt werden, dass sich das Schaumvermögen der erfindungsgemäßen Lactoside, Maltoside, Melibioside bei hohen Temperaturen (z.B. 50°C) auf dem gleichen Niveau wie bei 25°C befindet oder sich noch erhöht (Bsp 10-11). So sind Lac-EG-C12, Mal-EG-C12 und Mel-EG-C12 bei 50°C wesentlich bessere Schaumbildner als Natriumlauryethersulfat (SLES). Diese Eigenschaft ist sowohl nach sofortiger Bestimmung (immediate volume) als auch nach 30 Minuten zu beobachten (30 min. volume).
Dies ist insbesondere für kommerzielle Heißtemperaturanwendungen der erfindungsgemäßen Tenside interessant wie zum Beispiel für Waschpulver, Flüssigwaschmittel, Textilwaschpulver oder -pellets, Maschinengeschirrspülmittel oder auch für Heißgetränke (die z.B. schäumen sollen, z.B. Capucchino) oder als Lebensmittel, die in der Hitze hergestellt oder behandelt werden. Ferner sind die erfindungsgemäßen Tenside als Zubereitungen für ein heißes Schaumbad interessant.

Ferner können die erfindungsgemäß substituierten Disaccharide auch mit Glycerin als Spacer und einer hydrophoben Gruppe umgesetzt werden.

Beispielsweise kann man aus Maltose und Ethylhexylglycerin das entsprechende Maltosid herstellen (siehe später Kapitel *Schaumverhalten auf Haar*)

Die Maltoside können beispielsweise mit Lactosiden vorteilhaft gemischt werden, wie beispielsweise **Mal-EHG** mit **Lac-PG-C12.**

Weitere erfindungsgemäße Verbindungen sind (2'-Hexadecyloxy-ethyl) 4-O-(β-D-galactopyranosyl)-β-D-glucopyranosid, (2'-Dodecyloxy-ethyl) 4-O-(α-D-glucopyranosyl)-β-D-glucopyranosid, (2'-Tetradecyloxy-ethyl) 4-O-(α-D-glucopyranosyl)-β-D-glucopyranosid, (2'-Hexadecyloxy-ethyl) 4-O-(α-D-glucopyranosyl)-β-D-glucopyranosid, (2'-Dodecyloxy-ethyl) 6-O-(α-D-galactopyranosyl)-β-D-glucopyranosid, (2'-Tetradecyloxy-ethyl) 6-O-(α-D-galactopyranosyl)-β-D-glucopyranosid, (2'-Hexadecyloxy-ethyl) 6-O-(α-D-galactopyranosyl)-β-D-glucopyranosid, (2'-Dodecyloxy-propyl) 4-O-(α-D-glucopyranosyl)-β-D-glucopyranosid, (2'-Hexadecyloxy-ethyl) 4-O-(β-D-galactopyranosyl)-α-D-glucopyranosid, (2'-Dodecyloxy-ethyl) 4-O-(α-D-glucopyranosyl)-α-D-glucopyranosid, (2'-Tetradecyloxy-ethyl) 4-O-(α-D-glucopyranosyl)-α-D-glucopyranosid, (2'-Hexadecyloxy-ethyl) 4-O-(α-D-glucopyranosyl)-α-D-glucopyranosid, (2'-Dodecyloxy-ethyl) 6-O-(α-D-galactopyranosyl)-α-D-glucopyranosid, (2'-Tetradecyloxyethyl) 6-O-(α-D-galactopyranosyl)-α-D-glucopyranosid, (2'-Hexadecyloxy-ethyl) 6-O-(α-D-galactopyranosyl)-α-D-glucopyranosid, (2'-Dodecyloxy-ethyl) 4-O-(β-D-glucopyranosyl)-α-D-glucopyranosid, (2'-Dodecyloxy-propyl) 4-O-(α-D-glucopyranosyl)-α-D-glucopyranosid, (2'-Dodecyloxy-propyl) 6-O-(α-D-galactopyranosyl)-α-D-glucopyranosid, 1-*O*-(Dodecyloxy)-3-*O*-(4'-*O*-(β-D-galactopyranosyl)-β-D-glucopyranosyl)-glycerin, 1-*O-*(Dodecyloxy)-2-*O*-(4'-*O*-(β-D-galactopyranosyl)-β-D-glucopyranosyl)-glycerin, 1-*O-*(Dodecyioxy)-3-*O*-(4'-*O*-(β-D-gaiactopyranosyi)-α-D-giucopyranosyl)-glycerin, 1-*O-*(Dodecyloxy)-2-*O*-(4'-*O*-(β-D-galactopyranosyl)-α-D-glucopyranosyl)-glycerin, 1-O-(Dodecyloxy)-3-*O*-(4'-*O*-(α-D-glucopyranosyl)-β-D-glucopyranosyl)-glycerin, 1-*O-*(Dodecyloxy)-2-*O*-(4'-*O*-(α-D-glucopyranosyl)-β-D-glucopyranosyl)-glycerin,1-O-(Dodecyloxy)3-*O*-(4'-*O*-(α-D-glucopyranosyl)-α-D-glucopyranosyl)-glycerin, 1-*O-*(Dodecyloxy)2-*O*-(4'-*O*-(α-D-glucopyranosyl)-α-D-glucopyranosyl)-glycerin, (2'-(2"-Dodecyloxyethyloxy)-ethyl) 4-O-(β-D-galactopyranosyl)-β-D-glucopyranosid , (2'-(2"-(2"'-Dodecyloxyethyloxy)-ethyloxy)-ethyl) 4-O-(β-D-galactopyranosyl)-β-D-glucopyranosid, (2'-(2"-(2"'-(2""-Dodecyloxyethyloxy)-ethyloxy)-ethyloxy)-ethyl) 4-*O*-(β-D-galactopyranosyl)-β-D-glucopyranosid, (2'-(2"-Dodecyloxyethyloxy)-ethyl) 4-*O*-(β-D-galactopyranosyl)-α-D-glucopyranosid, (2'-(2"-(2"'-Dodecyloxyethyloxy)-ethyloxy)-ethyl) 4-*O*-(β-D-galactopyranosyl)-α-D-glucopyranosid, (2'-(2"-(2"'-(2""-Dodecyloxyethyloxy)-ethyloxy)-ethyloxy)-ethyl) 4-*O*-(β-D-galactopyranosyl)-α-D-glucopyranosid, 1-*O-*(Ethylhexyl)-3-*O*-(4'-*O*-(α-D-glucopyranosyl)-β-D-glucopyranosyl)-glycerin, 1-*O-*(Ethylhexyl)-2-*O*-(4'-*O*-(α-D-glucopyranosyl)-β-D-glucopyranosyl)-glycerin, 1-*O-*(Ethylhexyl)-3-*O*-(4'-*O*-(α-D-glucopyranosyl)-α-D-glucopyranosyl)-glycerin, 1-*O-*(Ethylhexyl)-2-*O*-(4'-*O*-(α-D-glucopyranosyl)-α-D-glucopyranosyl)-glycerin, 1-*O-*(Ethylhexyl)-3-*O*-(4'-*O*-(β-D-galactopyranosyl)-β-D-glucopyranosyl)-glycerin, 1-*O-*(Ethylhexyl)-2-*O*-(4'-*O*-(β-D-galactopyranosyl)-β-D-glucopyranosyl)-glycerin, 1-*O-*(Ethylhexyl)-3-*O*-(4'-*O*-(β-D-galactopyranosyl)-α-D-glucopyranosyl)-glycerin, 1-*O-*(Ethylhexyl)-2-*O*-(4'-*O*-(β-D-galactopyranosyl)-α-D-glucopyranosyl)-glycerin.
Erfindungsgemäß ist auch ein Tensid- oder Emulgatorsystem system aus (a) Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat, (b) Alkylsulfaten, beispielsweise Natrium-, Ammonium- und TEA-Laurylsulfat, (c) Natriumalkylsulfaten wie Natriumcetearylsulfat und mindestens einer erfindungsgemäßen Verbindung, besonders bevorzugt aus Laurylethersulfat und/oder Laurylsulfat und mindestens einer erfindungsgemäßen Verbindung.
Erfindungsgemäß ist auch eine kosmetische Zubereitung wie Haarwaschmittel, Haarkonditioniermittel, Frisiermittel, Zahnpflegemittel, Duschgel, Duschschaum, Badeschaum, Syndetstück, Haarfärbemittel, Gesichtsreiniger, flüssiges Handwaschmittel, Flüssigseife, Rasierhilfsmittel (Gel oder Schaum), Make-Up-Entferner, Tränkungsmedium für Tücher und/oder Gewebe enthaltend eine erfindungsgemäße Verbindung oder enthaltend ein erfindungsgemäßes Tensidsystem.
Erfindungsgemäß ist auch ein Reinigungsmittel für den Haushalt oder für technische Zwecke wie Haushaltsreiniger, Fensterreiniger, Flüssigwaschmittel für Textilien, Waschpulver für Textilien, Textilwaschpulveragglomerat oder -pellet, Maschinengeschirrspülmittel, Handgeschirrspülmittel enthaltend eine erfindungsgemäße Verbindung oder enthaltend ein erfindungsgemäßes Tensidsystem.
Erfindungsgemäß ist auch eine Lebensmittelzubereitung wie Bier, Getränk, Lebensmittel, Tofu enthaltend eine erfindungsgemäße Verbindung oder enthaltend ein erfindungsgemäßes Tensidsystem.

Erfindungsgemäß ist auch eine schaumförmige oder schäumbare Zubereitung einthaltend eine erfindungsgemäße Verbindung oder enthaltend ein erfindungsgemäßes Tensidsystem.
Bei all den erfindungsgemäßen Zubereitungen ist es besonders bevorzugt, wenn die Einsatzkonzentration der erfindungsgemäßen Verbindung 0,3 bis 25 Gew.% ist.
Erfindungsgemäß ist auch eine Verwendung einer erfindungsgemäßen Verbindung oder eines erfindungsgemäßen Tensidsystems als Tensid oder Cotensid in einem Haushaltsreiniger, Fensterreiniger, Flüssigwaschmittel für Textilien, Waschpulver für Textilien, Textilwaschpulveragglomerat oder -pellet, Maschinengeschirrspülmittel, Handgeschirrspülmittel, einem Haarwaschmittel, Haarkonditioniermittel, Frisiermittel, Zahnpflegemittel, Duschgel, Duschschaum, Badeschaum, Syndetstück, Haarfärbemittel, Creme, Lotion, Gesichtsreiniger, flüssiges Handwaschmittel, Flüssigseife, Rasierhilfsmittel (Gel oder Schaum), Make-Up-Entferner, Tränkungsmedium für Tücher und/oder Gewebe. Erfindungsgemäß ist auch eine Verwendung einer erfindungsgemäßen Verbindung oder eines erfindungsgemäßen Tensidsystems zur Herstellung eines Arzneimittels.
Erfindungsgemäß ist auch eine Verwendung einer erfindungsgemäßen Verbindung oder eines erfindungsgemäßen Tensidsystems zur Verbesserung der Schäumbarkeit einer wasserhaltigen Zubereitung.
Erfindungsgemäße Tensid- oder Emulgatorsysteme können zusätzlich Alkylamidopropylbetaine (z.B. Cocamidopropylbetain), Cocoamphoacetat, Laurylethersulfat, Fettsäure-Salze, Sarcosinate, Acylglutamate, Acyllactylate, Acyl-isethionate, Sulfosuccinate enthaltenden.
Erfindungsgemäße Zubereitungen können zusätzlich Polymere enthalten, wobei das Polymer/die Polymere nichtionisch, kationisch, anionisch oder amphoter sein können.
Die Erfindung umfassrt auch die Verwendung von ((2'-Dodecyloxy-ethyl) 4-O-(β-D-galactopyranosyl)-β-D-glucopyranosid) und/oder ((2'-Dodecyloxy-propyl) 4-O-(β-D-galactopyranosyl)-β-D-glucopyranosid) als Gelbildner und/oder als Schaumbildner für wässrige Systeme.
Die Erfindung umfasst auch ein Verfahren zur Herstellung einer erfindungsgemäßen Verbindung durch Umsetzung eines β-Peracetats der Komponente A mit einer Lewis Säure und dem Polyolether Aₓ-B.
Die Erfindung umfasst auch die Herstellung der erfindungsgemäßen Verbindungen durch Umsetzung eines β-Peracetats mitBortrifluorid-Diethyletherkomplex als Lewis-Säure, gemäß Beispiel 1, besonders bevorzugt mit einem α-Peracetat und einer verlängerten Reaktionszeit.
Die Erfindung umfasst auch ein Verfahren zur Herstellung einer erfindungsgemäßen Verbindung nach einem der vorangehenden Patentansprüche durch Umglycosylierung aus Butylglycosiden, Propylglycosiden, Ethylglycosiden, Methylglycosiden mit Polyolethern Aₓ-B.
Die Erfindung umfasst auch die Herstellung der erfindungsgemäßen Verbindungen auf dem Weg der direkten Fischer-Glycosylierung sowie die Herstellung der erfindungsgemäßen Verbindungen aus Glycosylhalogeniden, aus Glycosylimidaten, Orthoestern, n-Pentenyl-glycosiden, Thioglycosiden, Allyl-glycosiden, u.ä. bekannten Vorstufen.
Die Erfindung umfasst auch Laureth-2-Lactosid, Laureth-3-Lactosid, Laureth-2- Maltosid, Laureth-3- Maltosid sowie Produkte aus der Umsetzung eines Disaccharids mit einem Glycerinether und/oder einem Diglycerinether und/oder einem Triglycerinether, der Umsetzung von Lactose mit Ethylhexylglycerin, der Umsetzung von Lactose mit Laurylglycerinether, der Umsetzung von Maltose mit Ethylhexylglycerin sowie der Umsetzung von Maltose mit Lauryl-glycerinether.
Erfindungsgemäße Komponenten können weiterhin Tenside enthalten.

### A. Anionische Tenside

Vorteilhaft zu verwendende anionische Tenside sind:
Acylaminosäuren (und deren Salze), wie
   1. Acylglutamate, beispielsweise Dinatriumacylglutamat, Natriumacylglutamat und Natrium Caprylic / Capric Glutamat,
   2. Acylpeptide, beispielsweise Palmitoyl-hydrolysiertes Milchprotein, Natrium Cocoyl-hydrolysiertes Soja Protein und Natrium- / Kalium-Cocoyl-hydrolysiertes Kollagen, N-Cocoyl hydrolysiertes Weizenprotein, N-Cocoyl hydrolysierte Aminosäuren,
   3. Sarcosinate, beispielsweise Myristoyl Sarcosin, TEA-lauroyl Sarcosinat, Natriumlauroylsarcosinat und Natriumcocoylsarcosinat,
   4. Taurate, beispielsweise Natriumlauroyltaurat und Natriummethylcocoyltaurat,
   5. Acyllactylate, beispielsweise Lauroyllactylat, Caproyllactylat,
   6. Alaninate,
   7. Arginate, beispielsweise N-Lauroyl Arginat,
   8. Valinate, beispielsweise N-Lauroyl Valinat,
   9. Prolinate, beispielsweise N-Lauroyl Prolinat,
   10. Glycinat, beispielsweise N-Lauroyl Glycinat, Cocoamphopolycarboxyglycinat,
   11. Aspartate, beispielsweise N-Lauroyl Aspartate, Di-TEA-palmitoylaspartat,

### Carbonsäuren und Derivate, wie

1. Carbonsäuren und deren Alkali- und Erdalkalisalze, Triethanolaminsalze, oder Zink-Salze, beispielsweise Laurinsäure, Natrium-, Kalium-, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,
4. Natrium PEG-7 Olivenöl Carboxylat,

### Phosphorsäureester und Salze, wie beispielsweise DEA-Oleth-10-Phosphat und Dilaureth-4 Phosphat,

### Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat,
sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat,
3. Natriumalkylsulfate wie Natriumcetearylsulfat.

### B. Kationische Tenside

Vorteilhaft zu verwendende kationische Tenside sind:
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Dialkylethoxylatdimethylammoniumchloride oder -bromide wie beispielsweise Di (C14-C18) Alkyl (EO)3-20 dimethylammoniumsalze oder Distearyl(EO)5 dimethyl ammoniumchlorid und Distearyl(EO)15 dimethyl ammoniumchlorid. Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind:
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

Ferner können erfindungsgemäßen Zubereitungen auch kationische Polymere zugesetzt werden. Diese verbessern das Hautgefühl und können das Schaumverhalten der erfindungsgemäßen Rezepturen vorteilhaft beeinflussen.

### Kationische Polymere

1. Quaternäre Derivate von Celluloseethern. Bevorzugt ist beispielsweise Polyquaternium 10, Polyquaternium 24.
2. Quaternäre Derivate von Gummen. Bevorzugt ist beispielsweise Guar Hydroxypropyltrimonium chlorid und Hydroxypropyltrimonium hydroxypropyl guar.
3. Homopolymere und Copolymere von Dimethyl diallyammonium Chloride (DMDAAC).
   Bevorzugt ist beispielsweise Polyquaternium 4 und 6 und 7.
4. Homopolymere und Copolymere von Methacrylamidopropyl trimethyl ammonium chlorid (MAPTAC).
   Bevorzugt ist beispielsweise Polyquaternium 28 und Polymethacrylamidopropyltrimonium chlorid.
5. Homopolymere und Copolymere von Acrylamidopropyl trimethyl ammonium chlorid (APTAC).
6. Homopolymere und Copolymere von Methacryloyloxy ethyl trimethyl ammonium chlorid (METAC).
   Bevorzugt ist beispielsweise Polyquaternium 32 und Polyquaternium 37.
7. Homopolymere und Copolymere von Acryloyloxy ethyl trimethyl ammonium chlorid (AETAC).
   Bevorzugt ist beispielsweise Polyquaternium 33.
8. Homopolymere und Copolymere von Methacryloyloxy ethyl trimethyl ammonium methyl sulfat (METAMS).
   Bevorzugt ist beispielsweise Polyquaternium 5 und Polyquaternium 11 und Polyquaternium 14 und Polyquaternium 15 und Polyquaternium 36 und Polyquaternium 45.
9. Homopolymere und Copolymere von Vinylpyrrolidon. Bevorzugt ist beispielsweise Luviquat 905.
10. Kationische Polysaccharide, basierend auf Hydroxyalkylcellulosen, Stärken, Hydroxyalkylstärken, Polymere basierend auf Arabinose Monomeren, Polymere abgeleitet von der Xylose, Fucose, Fructose, Galacturonsäure und Glucuronsäure, Galactosamin und Glucosamin, Acetylglucosamin, Galactose, Mannose.
   Bevorzugt sind beispielsweise Hydroxypropyl trimethyl ammoniumchlorid Derivate von Stärke, die von Korn, Kartoffel, Rice, Tapioaca, Weizen und dergleichen sein kann.
11. Ammonium substituierte Polydimethylsiloxane.
   Bevorzugt sind beispielsweise Trimethylsilylamodimethicon (Dow Corning Q2-8220), Copolymere von Polydimethylsiloxan und Poly C2-C3 Alkylenether (Dow Corning 190, Dow Corning 2-5324, Dow Corning Q2-5220), Abil-Quat 3270.
12. PVP.
13. Polyethylenimine.
   Bevorzugt ist beispielsweise Lupasol FG (BASF), Lupasol G-35 (BASF), Lupasol P (BASF).
14. Chitosan.
15. Gellan Gum.
16. Polymere des Ethylenglycols (PEG-150 Distearat).
17. Blockcopolymere (PEG/PPG).
18. Cellulosen (Hydroxyethylcellulose, Cetylhydroxyethylcellulose, Hydroxypropylmethylcellulose, Natrium Carboxymethylcellulose usw.).
19. Polyacrylsäure und Derivate, Acrylsäure-Copolymere und Derivate, Alkyl-Acryate, Carbopole.
20. Carragenane und Derivate.
   Hyaluronsäure und Derivate.

Erfindungsgemäße Zubereitungen können auch Hautbefeuchtungsmittel (Moisturizer) enthalten wie Glycerin, Chitosan, Fucogel, Milchsäure, Propylenglycol, Dipropylenglycol, Butylenglycol, Mannitol, Natriumpyrolidoncarbonsäure, Hyaluronsäure und deren Salze, Aminosäuren wie Glycin, Harnstoff, Natrium und Kaliumsalze.

Ferner können auch vorteilhaft Salze für die erfindungsgemäßen Alkyl-Glycoside eingesetzt werden. Insbesondere vorteilhaft sind Calciumchlorid, Magnesiumsulfat, Natriumsulfat, KCl, Magnesiumchlorid und Kochsalz.
Es ist insbesondere auch vorteilhaft, wasserlösliche Wirkstoffe hinzuzufügen.

### Synthese

Die Herstellungsverfahren für erfindungsgemäße Komponenten sind dem Grunde nach bekannt (ViII, V., Böcker, T., Thiem, J., Fischer, F., Liq. Cryst., 6, 1989, 349ff.; v. Minden, H.M., Brandenburg, K., Seydel, U., Koch, M.H.J., Garamus, V.M., Willumeit, R., ViII, V., Chem. Phys. Lipids, 106, 2000, 157ff.). Diese liefern das Produkt in höchster Reinheit und nur einem Anomer nach Reinigung durch Säulenchromatographie. Die Ausbeute liegt, im Fall der Laurylethylenglycolglycoside, zwischen 30 und 45 %. Davon abweichend wurde gefunden, dass eine längere Reaktionszeit (18h statt 5h) im Fall der Ethylenglycol- und Propylenglycolether zu wesentlich höheren Ausbeuten führt. Die gewünschten Produkte konnten in Ausbeuten von 50 - 60 % erhalten werden, dabei wurde eine Erhöhung des Anteils an α-Anomer nicht beobachtet. Die Aufarbeitung kann jedoch vereinfacht werden, eine Säulenchromatographie ist dann nicht mehr erforderlich. Die Reaktion wird wie oben beschrieben nach der gewünschten Zeit durch Neutralisation abgebrochen. Salze werden mit Wasser ausgewaschen und die Reaktionsmischung wird dann zur Entfernung der Schutzgruppen im basischen Milieu bei pH 8-9 gerührt (NaOMe in MeOH). Nach Neutralisation mit saurem Ionentauscher wird das Lösungsmittel entfernt. Nicht umgesetzter Alkohol wird mit unpolarem Lösungsmittel (z.B. Petrolether 50-70) ausgewaschen, nicht umgesetzter Zucker wird mit polarem Lösungsmittel (z.B. Ethanol) ausgefällt. Die Ansatzgröße kann so wesentlich größer gewählt werden. Die so beschriebenen Verfahren liefern das β-Anomer. Die Darstellung des α-Anomers kann ähnlich erfolgen nur wird als Lewis-Säure Zinn(IV)-chlorid in äquimolarer Menge eingesetzt und die Reaktionszeit erhöht sich auf mind. 48 Stunden, die Ausbeute liegt im Fall der Laurylethylenglycolglycoside zwischen 25 und 30 %.

Der Einsatz von mehrwertigen Alkoholen oder Gemischen von Alkoholen führt zu Produktgemischen, die nach den beschriebenen Verfahren nicht mehr getrennt werden können. Dies ist aber in Anbetracht der Tatsache unnötig, daß die Bildung von Gemischen durchaus erwünscht ist und zu einer verbesserten Löslichkeit in Wasser führt. Die Umsetzung von Maltoseperacetat mit 1-Ethylhexyl-glycerin gibt das Maltosid als Mischung der 1,3- und und 1,2-substituierten Derivate des Glycerins in einer Gesamtausbeute von 44 %. Auch ist die Umsetzung von Laureth-2 (Fa. Sasol) mit Lactoseperacetat problemlos möglich. Man erhält ein Gemisch der Produkte, bedingt durch die inhomogene Zusammensetzung des eingesetzten Alkohols.
Die Herstellung der Verbindungen ist auch auf anderen bekannten Wegen möglich. So ist der Einsatz der o.g. Lewis-Säuren nicht zwingend erforderlich, es können auch andere für Glycosylierungen geeignete Lewis-Säuren (z.B TMS-Triflat) verwendet werden. Des weiteren ist auch eine Glycosylierung mit dem α-Peracetat oder einem Gemisch aus dem α- und β-Peracetat des Zuckers möglich, es verlängert sich dann entsprechend die Reaktionszeit.
Die Herstellung aus den Glycosylhalogeniden der Zucker ist ebenfalls möglich, auch auf anderen Wegen als der klassischen Königs-Knorr-Synthese. Es ist auch möglich die Verbindungen durch Umsetzung von zuvor hergestellten Glycosylimidaten, Thioglycosiden, n-Pentenyl-Glycosiden, Allyl-Glycosiden und ähnlichen aktivierten Zuckern herzustellen.

Von technischer Bedeutung ist die direkte Umsetzung des ungeschützten Zuckers mit dem Alkohol unter saurer Katalyse in der sogenannten Fischer-Glycosylierung.
Hierbei ist, wegen der besseren Ausbeuten, die Variante der Umglycosylierung aus den Butyl-, Propyl-, Ethyl- oder Methyl-Glycosiden zu bevorzugen.
Die Umsetzung eines kurzkettigen Glycosids liefert bei milderen Reaktionsbedingungen die gewünschten Produkte.

### Beispiele

### Herstellung von Tensiden (Lactosiden)

Bei den Laktosiden handelt es sich um α/β-Gemische. Folgende Lactoside wurden hergestellt:

### Beispiel 1

### Herstellung von Lac-EG-C12

6.8 g (10 mmol) Lactoseperacetat und 2.40 g (11 mmol) 2-Dodecyloxyethanol werden unter Stickstoffatmosphäre in 100 mL wasserfreiem Dichlormethan gelöst. 1.99 g (1.76 mL; 14 mmol) Bortrifluorid-diethyletherat werden zugetropft und die Lösung 5 Stunden bei Raumtemperatur gerührt. Nach vollständiger Umsetzung (DC) wird mit 50 mL gesättigter Natriumhydrogencarbonatlösung neutralisiert. Die organische Phase wird abgetrennt und die wässrige zweimal mit Dichlormethan extrahiert. Die vereinigten organischen Phasen werden zweimal mit Wasser gewaschen. Nach Trocknen über Magnesiumsulfat wird das Lösungsmittel unter vermindertem Druck entfernt. Der Rückstand wird säulenchromatographisch gereinigt (Petrolether 50-70 / Ethylacetat 1:1) und liefert das β-Produkt in guten Ausbeuten. Die acetylierte Verbindung wird in wasserfreiem Methanol gelöst und mit Natriummethanolat versetzt (pH 8-9). Danach wird bis zur vollständigen Umsetzung (DC) bei Raumtemperatur gerührt. Die Lösung wird mit Ionenaustauscher versetzt (Amberlyts IR 120, H⁺-Form), filtriert und das Lösungsmittel im Vakuum entfernt. Ausbeute: 1.6 g, entspr. 29 % d.Th.

### Beispiel 2

### Herstellung von Lac-PG-C12

Lactoseperacetat und Dodecyloxypropan-1-ol werden wie in *Beispiel 1* beschrieben umgesetzt, mit der Ausnahme, daß die Reaktionsdauer 18 Stunden beträgt. Anschließend wird neutralisiert und die Lösung bis zur Trockne eingeengt. Der Rückstand wird wie in *Beispiel 1* beschrieben entschützt und das β-Produkt säulenchromatographisch gereinigt (Chloroform / Methanol 3:1). Ausbeute: 3.2 g, entspr. 58 % d.Th.

### Beispiel 3

### Herstellung von Mal-EG-C12

6.8 g (10 mmol) Maltoseperacetat und 1.1 eq. (2.4 g; mmol) 2-Dodecyloxyethanol werden wie in Beispiel 2 beschrieben umgesetzt, mit der Ausnahme, das zuerst die Verbindung entschützt wird und das β-Produkt dann erst säulenchromatographisch (Chloroform / Methanol 3:1) gereinigt wird. Ausbeute: 3.3 g, entspr. 60 % d.Th.

### Beispiel 4

### Herstellung von Lac-PG-C12

34 g (50 mmol) Lactoseperacetat, 1.1 äq. (13.4 g; 55 mmol) 3-Dodecyloxypropan-1-ol, 1.4 äq. (9.9 g; 8.8 mL; 70 mmol) Bortrifluoriddiethyletherkomplex werden in 300 mL wasserfreiem Dichlormethan wie in Beispiel 2 beschrieben umgesetzt, mit der Ausnahme, daß auf eine säulenchromatographische Reinigung verzichtet wird. Stattdessen wird nicht umgesetzter Alkohol mit Petrolether (50-70) ausgewaschen, der verbleibende Rückstand wird dann in Ethanol aufgenommen und nicht umgesetzte Lactose wird abfiltriert. Das Lösungsmittel wird im Vakuum entfernt. Man erhält das Produkt als Gemisch der beiden Anomere. Ausbeute: 16.6 g, entspr. 60 % d.Th.

### Beispiel 5

### Herstellung von Lac-Laureth-2

34 g (50 mmol) Lactoseperacetat und 20 mL Laureth-11 (Marlipal 24/20, Fa. Sasol) werden wie in Beispiel 4 beschrieben umgesetzt. Das Produkt ist ein Gemisch der α- und β-Anomeren wobei der β-Anteil überwiegt. Die Produktverteilung hängt vom eingesetzten Alkohol ab. Typischerweise erhält man ein Gemisch der Lactoside des Lauryl- / Myristyl Ethylenglycols, Lauryl- / Myristyl Diethylenglycols, Lauryl- / Myristyl Triethylenglycols und höher ethoxylierter Addukte, wobei der Anteil der diethoxylierten überwiegt. Ausbeute: 25 g.

### Beispiel 6

### Herstellung von Mal-EHG

6.8 g (10 mmol) Maltoseperacetat und 4.1 g (4.3 mL; 20 mmol) (2-Ethylhexyl)-glycerin (Sensiva SC50, Fa. Schülke & Mayr) werden wie in Beispiel 1 beschrieben umgesetzt. Ausbeute: 2.3 g, entspr. 44 % d.Th.

### Beispiel 7

### Herstellung von Lac-α-EG-C12

6.8 g (10 mmol) Lactoseperacetat und 1.1 äq. (2.54 g; 11 mmol) 2-Dodecyloxyethanol werden unter Stickstoffatmosphäre in 100 mL wasserfreiem Dichlormethan gelöst. 10 mmol (2.6 g; 1.14 ml) Zinntetrachlorid werden innerhalb von 30 min. zugetropft. Die Lösung wird anschließend 72 Stunden bei Raumtemperatur gerührt. Die Reaktion wird durch Zugabe von gesättigter Natriumhydrogencarbonatlösung abgebrochen und die organische Phase wird abgetrennt. Die wässrige Phase wird einmal mit Dichlormethan extrahiert. Die vereinigten organischen Phasen werden mit Wasser gewaschen, über Magnesiumsulfat getrocknet und das Lösungsmittel unter vermindertem Druck entfernt. Der Rückstand wird in 50 mL wasserfreiem Methanol aufgenommen und wie in Beispiel 1 beschrieben entschützt. Das α-Anomer wird nach säulenchromatographische Trennung erhalten (Chloroform / Methanol 3:1). Ausbeute: 1.7 g, entspr. 30 % d.Th.

### Beispiele 7a

### Allgemeine Arbeitsvorschrift für die Herstellung von Octaacetyl-β-Lactose:

### Darstellung von 1,2,3,4-Tetra-O-acetyl-4-O-(2',3',4',5'-tetra-O-acetyl-β-D-galactopyranosyl)-β-D-glucopyranosid; (Octaacetyl-β-Lactose) Lac-OAc (β-Anomer)

40.0 g (0.117 mol) wasserfreie Lactose (bzw. Laktose-Monohydrat) wurden in 110 mL (1.17 mol) Essigsäureanhydrid gelöst. Das Gemisch wurde auf -5 °C gekühlt und es wurde eine Mischung aus 4 Tropfen konzentrierter Schwefelsäure in 1 mL Essigsäureanhydrid langsam und tropfenweise dazugegeben. Nach beendeter Zugabe wurde noch 10 Minuten bei -5°C gerührt, danach wurde 2 Stunden bei 60 °C gerührt. Die Lösung wurde auf Eis gegossen und mit Dichlormethan versetzt. Die Phasen wurden getrennt und die wässrige Phase wurde zweimal mit 50 mL Dichlormethan extrahiert.
Die vereinigten organischen Phasen wurden in 600 mL gesättigter Natriumhydrogencarbonat-Lösung getropft. Die Lösung wurde filtriert, um das überschüssige feste Natriumhydrogencarbonat zu entfernen. Die wässrige Phase wurde zweimal mit 50 mL Dichlormethan extrahiert. Die vereinigten organischen Phasen wurden mit 100 mL H₂O gewaschen, über MgSO₄ getrocknet und das Lösungsmittel wurde unter vermindertem Druck entfernt.
Das Produkt wurde in frisch destilliertem Ethanol umkristallisiert.

### Allgemeine Arbeitsvorschrift Laktoside aus Fettalkoholethoxylaten und Laktose: [Lutensol^{®} ON 30 (C10-Alkohol +3 EO); Berol 260 (C9-C11 Alkoholethoxylat (4)); Berol 532 (C11 Alkoholethoxylat (2)); Berol 533 (C11 Alkoholethoxylat (3))]:

6.8 g (10 mmol) 1,2,3,4-Tetra-*O*-acetyl-4-*O*-(2',3',4',5'-tetra-*O*-acetyl-β-D-galactopyranosyl)-β-D-glucopyranosid und (14 mmol) des jeweiligen Alkoholethoxylats [Lutensol^{®} ON 30 (C10-Alkohol +3 EO); Berol 260 (C9-C11 Alkoholethoxylat (4)); oder Berol 532 (C11 Alkoholethoxylat (2)); Berol 533 (C11 Alkoholethoxylat (3))] in 100 mL abs. Dichlormethan wurden unter Rühren gelöst. Hierzu wurden 1.99 g (14 mmol) Bortrifluorid-Etherat langsam zugetropft. Anschließend wurde die Reaktionsmischung über Nacht bei Raumtemperatur gerührt. Zur Aufarbeitung des Produktes wurden 100 mL gesättigte Natriumhydrogencarbonat-Lösung zugegeben und für weitere 30 min. gerührt. Die wässrige Phase wurde zweimal mit jeweils 30 mL Dichlormethan extrahiert, die vereinten organischen Phasen wurden zweimal mit 60 mL gesättigter Natriumhydrogencarbonat-Lösung und einmal mit 60 mL destilliertem Wasser gewaschen. Nach anschließender Trocknung über Magnesiumsulfat wurde das Lösungsmittel unter verminderten Druck entfernt. Das Produkt wurde säulenchromatographisch gereinigt (Petrolether (50-70), Ethylacetat 2:1).

### DC:

Laufmittel (Petrolether (50-70), Ethylacetat 1/1).

Die peracetylierten Laktoside wurden jeweils mit ca. 80 mL wasserfreien Methanol und 3 Spatelspitzen Natiummethylat (pH-Wert-Prüfung (pH: 10)) drei Stunden gerührt. Die Lösungen wurden nach Zugabe von Ionenaustauscher Dowex 50 WX 4 ca. 15 Minuten gerührt. Nach anschließender pH-Wert-Prüfung (pH: 4) wurde der Ionenaustauscher abfiltriert und das Lösungsmittel unter verminderten Druck entfernt. Die Produkte wurden säulenchromatographisch gereinigt (Chloroform, Methanol 3:1). Die Produkte sind zum Teil α/β-Gemische.

Hergestellt wurden nach der eben genannten allgemeinen Arbeitsvorschrift folgende Produkte 13 bis 16:

### Produkt 13.)

(2-[2-[2-(Decyloxy) ethoxy] ethoxy] ethyl)-4-*O*-(β-D-galactopyranosyl)-β-D-glucopyranosid
**(Lac-EG₃C10),**
und
(2-[2-[2-(Decyloxy) ethoxy] ethoxy] ethyl)-4-*O*-(β-D-galactopyranosyl)-α-D-glucopyranosid
**(Lac-α-EG₃C10),**

### Umsetzung von Lutensol^{®} ON 30 mit Lactose

Das **Lutensol^{®} ON 30 (C10-Alkohol +3 EO)** wurde von der Firma BASF Ludwigshafen, 67056 Deutschland bezogen. Man erhält eine Mischung von **Lac-EG₃C10 und Lac-α-EG₃C10**

### Produkt 14.)

(2-[2-[2-[2-(Nonyloxy) ethoxy] ethoxy] ethoxy)] ethyl)-4-*O*-(β-D-galactopyranosyl)-β-D-glucopyranosid
**(Lac-EG₄C9)**

(2-[2-[2-[2-(Nonyloxy) ethoxy] ethoxy] ethoxy)] ethyl)-4-*O*-(β-D-galactopyranosyl)-α-D-glucopyranosid
**(Lac-α-EG₄C9)**

(2-[2-[2-[2-(Decyloxy) ethoxy] ethoxy] ethoxy)] ethyl)-4-*O*-(β-D-galactopyranosyl)-β-D-glucopyranosid
**(Lac-EG₄C10)**

(2-[2-[2-[2-(Decyloxy) ethoxy] ethoxy] ethoxy)] ethyl)-4-*O*-(β-D-galactopyranosyl)-α-D-glucopyranosid
**(Lac-α-EG₄C10)**

(2-[2-[2-[2-(Undecyloxy) ethoxy] ethoxy] ethoxy)] ethyl)-4-*O*-(β-D-galactopyranosyl)- β-D-glucopyranosid
**Lac-EG₄C11**

(2-[2-[2-[2-(Undecyloxy) ethoxy] ethoxy] ethoxy)] ethyl)-4-*O*-(β-D-galactopyranosyl)-α-D-glucopyranosid
**(Lac-α-EG₄C11)**
**Laktosid: Lac-EG₄C9 und Lac-a-EG₄C9**
**Laktosid: Lac-EG₄C10 und Lac-α-EG₄C10**
**Laktosid: Lac-EG₄C11 und Lac-α-EG₄C11**

### Umsetzung von Berol 260 (Akzo) mit Lactose

Das **Berol 260 (C11 Alkoholethoxylat (4)** wurde von der Firma Akzo Nobel Surface Chemistry AB SE 44485 Stenungsund, Sweden bezogen. Man erhält hauptsächlich eine Mischung von **Lac-EG₄C9, Lac-α-EG₄C9, Lac-EG₄C10, Lac-α-EG₄C10, Lac-EG₄C11 und Lac-α-EG₄C11.**

### Produkt 15.)

**Laktosid: Lac-EG₂-C11 und Lac-α-EG₂-C11**

(2-[2-(Undecyloxy) ethoxy] ethyl)-4-*O*-(β-D-galactopyranosyl)-β-D-glucopyranosid
**(Lac-EG₂C11)**

(2-[2-(Undecyloxy)ethoxy] ethyl)-4-*O*-(β-D-galactopyranosyl)-α-D-glucopyranosid
**(Lac-α-EG₂C11)**

### Umsetzung von Berol 532 mit Lactose.

Das **Berol 532 (C11 Alkoholethoxylat (2))** wurde von der Firma Akzo Nobel Surface

Chemistry AB SE 44485 Stenungsund, Sweden bezogen. Man erhält hauptsächlich eine Mischung von **Lac-EG₂-C11 und Lac-α-EG₂-C11.**

### Produkt 16.)

### Laktosid: Lac-EG₃-C11 und Lac-α-EG₃-C11

(2-[2-[2-(Undecylyoxy) ethoxy] ethoxy] ethyl)-4-O-(β-D-galactopyranosyl)-β-D-glucopyranosid
**(Lac-EG₃C11)**

(2-[2-[2-(Undecyloxy) ethoxy] ethoxy] ethyl)-4-*O*-(β-D-galactopyranosyl)-α-D-glucopyranosid
**(Lac-α-EG₃C11)**

### Umsetzung von Berol 533 (C11 Alkoholethoxylat (3)) mit Lactose

Das **Berol 533 (C11 Alkoholethoxylat (3))** wurde von der Firma Akzo Nobel Surface Chemistry AB SE 44485 Stenungsund, Sweden bezogen. Man erhält hauptsächlich eine Mischung von **Lac-EG₃-C11 und Lac-α-EG₃-C11.**

### Schnelltest zur Untersuchung des Schaumverhaltens:

Die Messungen wurden in 5 mL Präparategläsern durchgeführt. Lösungen werden durch Einwiegen von 0.05 g Tensid und Zugabe von 0,95 g bedestillierten Wasser bis zu einem Gesamtgewicht von 1 g (ca. 1 ml) hergestellt. Zur Schaumbildung wird das Präparateglas 20-mal innerhalb von 10 sec geschüttelt. Die Schaumvolumina werden direkt nach dem Schütteln der Präparategläser (Schaumungsvermögen) und nach 20 Minuten (Schaumstabilität) bestimmt.

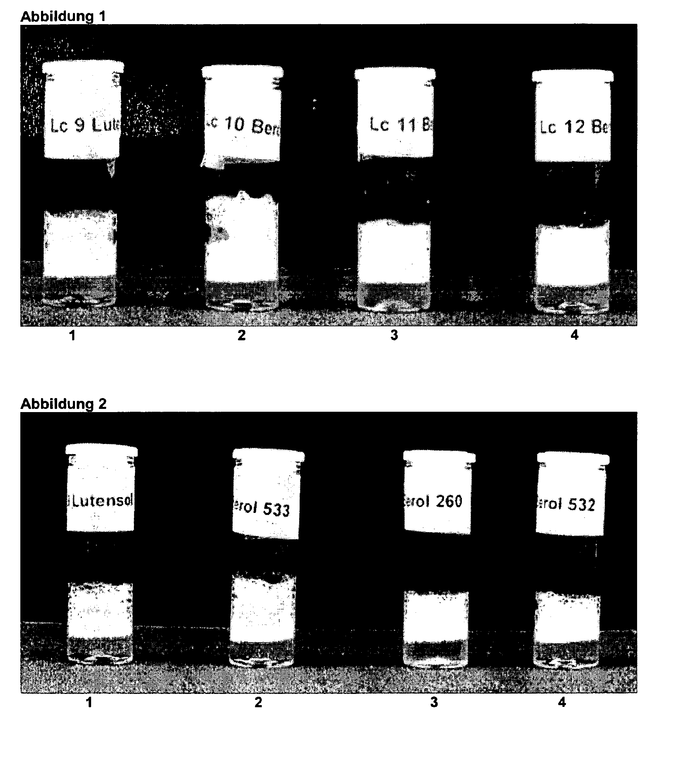

**Abbildung 1** zeigt den Schaum direkt nach dem Schütteln. Gezeigt sind Laktoside aus Laktose und entsprechendem ethoxylierten Fettalkohol. Dabei sind 1.) Lutensol^{®} ON 30 (C10-Alkohol +3 EO), 2.) Berol 533 (C11 Alkoholethoxylat (3)), 3.) Berol 260 (C9-C11 Alkoholethoxylat (4)), 4.) Berol 532 (C11 Alkoholethoxylat (2))

**Abbildung 2** zeigt den Schaum nach 20 Minuten. Gezeigt sind Laktoside aus Laktose und entsprechendem ethoxyliertem Fettalkohol. 1.) Lutensol^{®} ON 30 (C10-Alkohol +3 EO), 2.) Berol 533 (C11 Alkoholethoxylat (3)), 3.) Berol 260 (C9-C11 Alkoholethoxylat (4)), 4.) Berol 532 (C11 Alkoholethoxylat (2))

### Ergebnisse:

Die Laktoside 1-4 zeigen bei dieser Konzentration sehr gute Schaumeigenschaften. Das Schaumvermögen ist bei allen sehr gut ausgeprägt. Der Schaum ist optisch feinporig. Nach 20 min zeigen sich kaum Veränderungen des Schaumbildes, dies spricht für eine ausgeprägte Schaumstabilität.

### Materialen:

Als Edukte wurden die folgenden Substanzen eingesetzt:
Als Disaccharid wurde das Laktose-Monohydrat **Variolac 99 edible G800, der Firma Biolac GmbH & Co. KG, 31097 Harbarnsen** verwendet.
Zur Herstellung der Verbindungen kann sowohl Laktose-Monohydrat, bzw. wasserfreie Laktose, verwendet werden.

### Gelbildung durch erfindungsgemäße Tenside:

Die Gelbildung der Laurylglycol-Verbindungen **Lac-EG-C12** und **Lac-PG-C12** ist insofern ungewöhnlich, als daß die erfindungsgemäßen Verbindungen keine Struktur aufweisen, die normalerweise für Gelbildner üblich sind. Dementsprechend ist die Gelbildung in hohem Maße von dem Verhältnis zwischen den hydrophilen und hydrophoben Molekülteilen abhängig. Der Einsatz eines Spacers, bei der Verbindung **Lac-EG-C12** Ethylenglycol und bei **Lac-PG-12** Propylenglycol, führt zur Bildung eines stabilen Gels.
Besonders vorteilhaft sind dabei die Substanzen **Lac-EG-C12** und **Lac-PG-C12.** So bildet z.B. **Lac-EG-C12** bei einer Konzentration von 0.1 Gew.% ein Gel bis zu einer Übergangstemperatur von 43 °C und **Lac-PG-C12** bei 0.1 Gew.% bis 41 °C. Oberhalb dieser Übergangstemperatur wird das Gel flüssig. Die Struktur **Lac-EG-C12** ist darüber hinaus besonders vorteilhaft als Gelbildner, da das Gel mechanisch stabiler ist. Eine genauere Aufstellung der Übergangstemperaturen bei verschiedenen Konzentrationen findet sich in Beispiel 1 und Abbildung 2. Für medizinische Anwendungen weiter interessant ist die Gelbildung in Mischungen von Wasser und Dimethylsulfoxid (DMSO). Auch hier erweist sich die Verbindung **Lac-EG-C12** als besonders vorteilhaft. Das Gelbildungsverhalten ist expemplarisch für eine Konzentration von 0.1 Gew.% in Tabelle 2 aufgeführt.
Des Weiteren wurde gefunden, daß **Lac-EG-C12** auch in Mischungen mit anderen Tensiden, wie z.B. Laurylethersulfat und Cotensiden, wie z.B. Cocoamphoacetat, Cocamidoproyl Betain, Cocoylglutamat in der Lage ist, Gele zu bilden.

Der Fachmann weiß, dass in klassischen Tensidsystem auf Lauryethersulfat-Basis durch Zusatz von hohen Konzentrationen an Kochsalz die Viskosität erhöht wird oder durch Zusatz von wasserlöslichen Polymeren oder durch beides.
Ferner werden bei reinen Tensidformulierungen, die APGs enthalten und frei von Aniontensiden wie zum Beispiel Laurylethersulfat sind, Verdicker zugesetzt, da APGs in geringen Konzentrationen keine verdickende Wirkung haben.

### Beispiel 8

### Gelbildung von Lac-EG-C12 in Wasser

**Tabelle 1: Phasenumwandlungstemperaturen (T_{gel}) vom Gel zur isotropen Lösung für unterschiedliche Konzentrationen an Lipid (Abweichung ± 0.5 °C). Die Verbindung bildet ein stabiles Gel und oberhalb von T_{gel} eine klare Lösung. T_{gel} ist definiert als die Temperatur bei der das Gel sich beginnt aufzulösen. Die Temperaturspanne des Übergangs beträgt im Normalfall 2 bis 5 °C.**

| Konzentration | Konzentration | **T_{gel}** |
|---|---|---|
| **Gew.%** | **mM** | **[°C]** |
| 10 | 180.40 | 51 |
| 5 | 90.20 | 51 |
| 1 | 18.04 | 50 |
| 0.5 | 9.02 | 48 |
| 0.2 | 3.61 | 46 |
| 0.13 | 2.41 | 45 |
| 0.1 | 1.80 | 43 |
| 0.08 | 1.44 | 40 |
| 0.07 | 1.20 | 38 |
| 0.06 | 1.03 | 35 |
| 0.05 | 0.90 | 30 |
| 0.04 | 0.72 | 21 |
| 0.03 | 0.60 | 10 |

Phasendiagramm der Verbindung **Lac-EG-C12** in Wasser.
(siehe Abb. 1)

### Beispiel 8a

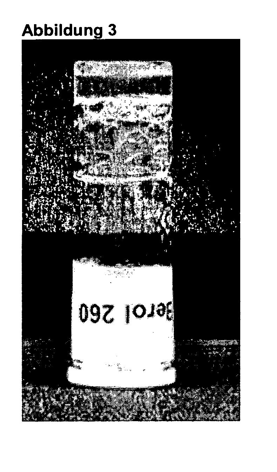

Das Laktosid aus Berol 260 und Lactose (Produkt 14)) ist zur Gelbildung befähigt. Das resultierende Gel ist zum Beispiel bei einem Gehalt von 5 Gew.% in Wasser aussergewöhnlich klar. **Siehe** **Abbildung 3****.**

### Beispiel 9

### Gelbildung von Lac-EG-C12 in Dimethylsulfoxid (DMSO) / Wasser - Mischungen

**Tabelle 2: Phasenumwandlungstemperaturen (T_{gel}) vom Gel zur isotropen Lösung für unterschiedliche Lösungsmittelzusammensetzung (Dimethylsulfoxid, DMSO und Wasser, H₂O) bei gleichbleibender Lipidkonzentration (0.1 Gew.%) (Abweichung ± 0.5 °C). Die Verbindung formt klare Gele oder geliert nur teilweise, oberhalb der Übergangstemperatur bildet sie eine klare Lösung. T_{gel} ist definiert als die Temperatur bei der das Gel sich beginnt aufzulösen. Die Temperaturspanne des Übergangs beträgt im Normalfall 2 bis 5 °C.**

| G=Gel | | | | | |
|---|---|---|---|---|---|
| Gₚ = Teilweise geliert | | | | | |
| I = Isotrope Lösung | | | | | |
| S = Fest beim Abkühlen (bedingt durch gefrorenes Lösungsmittel) | | | | | |
| **H₂O /DMSO** | **-27 °C** | **4 °C** | **20 °C** | **39 °C** | **50 °C** |
| **9:1** | S | G | G | G | I |
| **8:2** | S | G | G | G | I |
| **7:3** | S | G | G | G | I |
| **6:4** | S | G | G | G | I |
| **5:5** | G | G | G | G | I |
| **4:6** | S | G | G | Gₚ | I |
| **3:7** | S | Gₚ | I | -- | -- |
| **2:8** | S | I | I | -- | -- |
| **1:9** | S | I | I | -- | -- |

### Tenside / Tensidmischungen ohne Gelbildung

Die Gelbildung kann auch durch Beimischung anderer Verbindungen aufgehoben werden. Dies kann erreicht werden indem man Mono-, Di- und Triethylenglycolalkylether Lactoside miteinander mischt.

Als besonders vorteilhaft erweisen sich dabei die Gemische, die man durch Umsetzung von Laureth-2 bzw. Laureth-3 mit Lactose **(Lac-Laureth-2** und **Lac-Laureth-3)** erhält. Des Weiteren ist das α-Anomer des **Lac-EG-C12** besonders bevorzugt, da es ebenfalls kein Gel bildet. Darüber hinaus wurde gefunden, daß ab einem Anteil an α-Anomer in einer Konzentration von mehr als 80 Gew.% die Gelbildung durch das β-Anomer **Lac-EG-C12** aufgehoben wird. Auch wurde beobachtet, daß eine Mischung aus 13 Gew%. Laurylethersulfat mit 2 Gew.% des β-Anomers von **Lac-EG-C12** kein Gel mehr bildet, bei einem Verhältnis von 11 Gew.% Laurylethersulfat zu 4 Gew.% des β-Anomers von **Lac-EG-C12** die Gelbildung aber wieder einsetzt. Auf diese Weise ist es möglich, die Gelbildung durch das Verhältnis (und die Auswahl) von Lactosid zu Cotensid zu steuern.

### Schaumverhalten wässriger Zubereitungen mit erfindungsgemäßen Tensiden

Einige der Substanzen wurden exemplarisch auf ihre Schaumeigenschaften in Wasser hin untersucht.

### Methode

Messungen werden in 100 mL Meßzylindern durchgeführt. Vor Benutzung wird der Meßzylinder mit konzentrierter Nitriersäure gefüllt und 24 Stunden gelagert, danach mit bidestilliertem Wasser für weitere 24 Stunden. Lösungen werden durch Einwaage des Lipids und Zugabe von bidestilliertem Wasser bis zu einem Gesamtgewicht von 10 g (ca. 10 mL) hergestellt. Lösungen der jeweils niedrigeren Konzentration werden durch Verdünnung der ersten hergestellt. Vor der Messung wird der Meßzylinder bei der gewünschten Temperatur gelagert. Zur Schaumbildung wird der Meßzylinder 20 mal innerhalb von 30 Sekunden umgeschwenkt. Die Messung wird bis zu einer Abweichung von nicht mehr als 5 % wiederholt. Schaumvolumina werden direkt nach dem Schwenken des Zylinders (Schäumungsvermögen) und nach 30 Minuten (Schaumstabilität) bestimmt. '

### Ergebnisse

Sehr gute Schaumeigenschaften im Vergleich zu z.B. ionischen Tensiden (z.B. Natriumlaurylethersulfat, SLES oder Natriumlaurylsulfat, SDS) wurden ermittelt, insbesondere bei höherer Temperatur ist das Schäumungsvermögen und die Stabilität der Schäume deutlich besser.

Bei Erniedrigung der Konzentration um Faktor 10, ist bei ionischen Tensiden faktisch keine Schaumbildung mehr festzustellen, hingegen zeigen die untersuchten Lipide immer noch ein deutliches Schaumbildungsvermögen.

### Beispiel 10

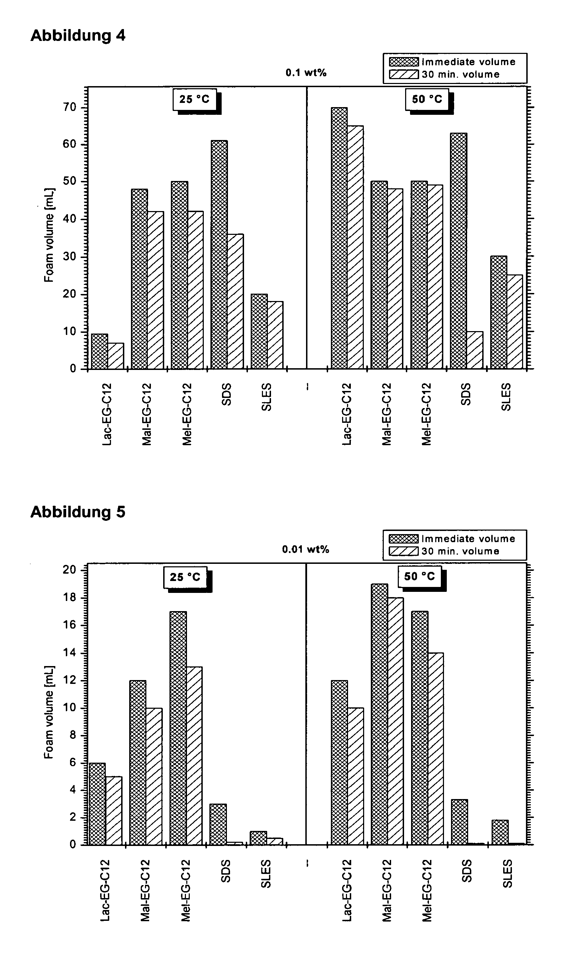

Schaumvermögen und Schaumstabilität ausgewählter Verbindungen bei einer Tensidkonzentration von 0.1 Gew.%, und einer Temperatur von 25 °C bzw. 50 °C **(Lac-EG-C12:** Laurylethylenglycol-β-Lactosid, **Mal-EG-C12:** Laurylethylenglycol-β-Maltosid, **Mel-EG-C12:** Laurylethylenglycol-β-Melibiosid, **SDS:** Natrium Dodecylsulfat, **SLES:** Laurylethersulfat).
**Siehe** **Abbildung 4****.**

### Beispiel 11

Schaumvermögen und Schaumstabilität ausgewählter Verbindungen bei einer Tensidkonzentration von 0.01 Gew.%, und einer Temperatur von 25 °C bzw. 50 °C **(Lac-EG-C12:** Laurylethylenglycol-β-Lactosid, **Mal-EG-C12:** Laurylethylenglycol-β-Maltosid, **Mel-EG-C12:** Laurylethylenglycol-β-Melibiosid, **SDS:** Natrium Dodecylsulfat, **SLES:** Laurylethersulfat).
**Siehe** **Abbildung 5****.**

### Schaumverhalten auf Haar

Eine weitere wichtige Eigenschaft ist das Schäumungsvermögen auf Haut oder Haar. Die Daten der Ross-Miles Schaumtests sind oftmals nicht mit der Schaumbildung auf Haar und Haut korrelierbar.

Hier zeigte sich erstaunlicherweise das die erfindungsgemäßen Verbindungen eine vergleichbare Schaumbildung auf der Haut oder dem Haar wie z.B. Laurylethersulfat zeigen, jedoch in deutlich niedrigeren Einsatzkonzentrationen. Aus den Daten für Lac-EG-C12 (Tab. 10) war nicht unbedingt zu erwarten, dass es sich in Wasser beim Verreiben des Tensids in der Hand um einen guten Schaumbildner handelt. Auch in Gegenwart von Haaren wird beim Verreiben des Tensids eine Schaumbildung beobachtet. Dazu wurde 1 Gew% der erfindungsgemäßen Substanz in Wasser in Gegenwart von Haarsträhnen gelöst und in der Hand verrieben.
Der Schaum ist wesentlich feinporiger als vergleichbare Schäume von Laurylethersulfat. Als bester Schaumbildner in Form seiner erfindungsgemäßen Einzelkomponente erwies sich das β-Anomere **Lac-EG-C12** bzw. auch **Lac-Laureth-2** bzw. **Lac-Laureth-3.**

Das Schaumverhalten der weiteren erfindungsgemäßen Tenside läßt sich jedoch in Schaumvermögen und Feinporigkeit des Schaum durch Zugabe von klassischen Cotensiden, wie z.B. Laurylethersulfat, Betainen, Amphoacetaten und Acyl-Glutamaten steuern. Hierbei zeigte sich, das z.B. bei Mischungen aus Lactosiden des Laureth-2 und Laureth-3 der Zusatz von 0.5 und 1 Gew.% Cotensid (Natrium Cocoamphoacetat, Cocamidopropylbetain, Dinatrium Cocoylglutamat, bei 3 Gew.% Gesamtgehalt) das Schaumverhalten verbessert.

Ein erfindungsgemäßes Cotensid ist das Maltose Glycosid des Ethylhexylglycerins. Zuckerderivate des 1-O-(2-Ethylhexyl)-glycerins sind noch unbekannt. Nur die Verwendung des 1-*O*-(2-Ethylhexyl)-glycerin in Körperpflegeprodukten ist in DE414047, DE4240674, EP599433 und US5516510 beschrieben. Als besonders bevorzugt als Cotenside haben sich Glycoside des 1-*O*-(2-Ethylhexyl)-glycerins auf Basis von Maltose erwiesen. Sowohl das Glycosid an der primären und an der sekundären Hydroxygruppe des Glycerins, als auch das Gemisch verstärken die Schaumbildung der erfindungsgemäßen Verbindungen. Auch die Stabilität der Schäume und die Feinporigkeit werden erhöht. Als besonders vorteilhaft erwies sich eine 1:1 Mischung aus **Lac-PG-C12** und dem hier beschriebenen Cotensid **Mal-EHG.**
Des Weiteren führt der Einsatz der erfindungsgemäßen Tenside als Cotenside in Mischungen mit Laurylethersulfat zu einer Verbesserung der Schaumeigenschaften bzw. erlaubt es, die Hautverträglichkeit im Vergleich zu einer Formulierung, die nur Laurylethersulfat enthält, zu verbessern.

### Beispiel 12

Schaumbild und Größenverteilung der Gasbläschen nach 0 min. (Abb. 2 und 3) und nach 10 min. (Abb. 4 und 5) von **Lac-EG-C12** in einer Gesamtkonzentration von 1 Gew.%. in Wasser. Man erkennt, daß der Schaum zu Beginn fast ausschließlich aus Bläschen mit einer Porengröße von unter 10000 µm² besteht. Nach 10 min. wird die Größenverteilung etwas flacher, wobei immer noch rund ein Viertel der Bläschen eine Größe von unter 10000 µm² aufweist.

### Beispiel 13

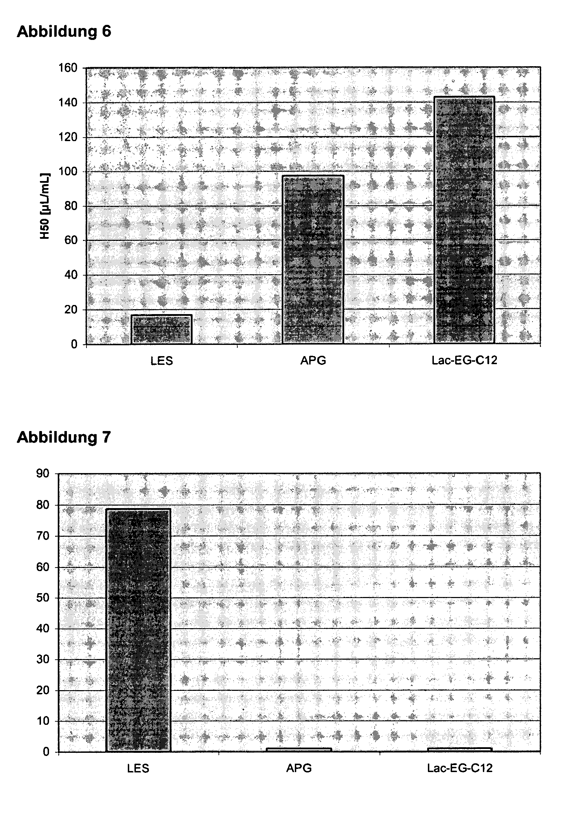

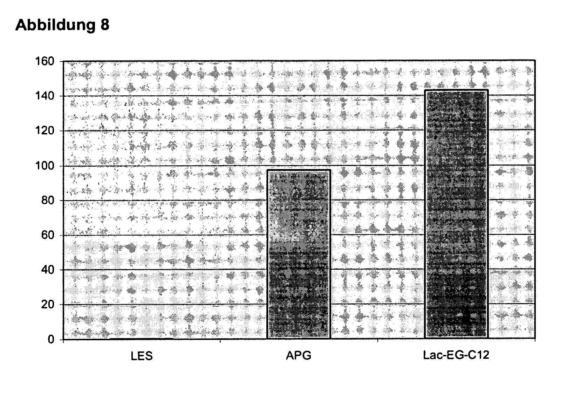

Schaumbild und Größenverteilung der Gasbläschen nach 0 min. (Abb. 6 und 7) und nach 10 min. (Abb. 8 und 9) einer Mischung aus **Lac-PG-C12 / Mal-EHG** im Verhältnis 1:1 und einer Gesamtkonzentration von 1 Gew.%. in Wasser. Man erkennt in diesem Beispiel, das ebenfalls wie im vorangehenden Beispiel, zu Beginn ein sehr feinporiger Schaum gebildet wird, in dem nach 10 min. ebenfalls die Bläschen mit einer Größe von unter 10000 µm² dominieren.

### Beispiel 14

Schaumbild und Größenverteilung der Gasbläschen nach 0 min. (Abb. 10 und 11) und nach 10 min. (Abb. 12 und 13) einer Laurylethersulfat enthaltenden Tensidmischung in einer Gesamtkonzentration von 15 Gew.%. in Wasser. Im Gegensatz zu den beiden vorherigen Beispielen, sieht man daß bereits der sofort gebildete Schaum eine sehr breite Größenverteilung der Gasbläschen aufweist, somit also grobporiger ist. Nach 10 min. hat sich die Größenverteilung sogar zu Größen von über 100000 µm² hin verschoben.

### Verträglichkeit

Überraschender Weise zeichnen sich einige der erfindungsgemäßen Verbindungen durch eine deutlich bessere Hautverträglichkeit und eine wesentlich geringere Reizung der Schleimhaut im Vergleich zu Laurylethersulfat, Laurylsulfat und sogar APG aus. Die Verträglichkeitsuntersuchungen erfolgte nach literaturbekannten Methoden (Pape, W., Pfannenbecker, U., Hoppe, U.: Validation of the red blood cell test as an in vitro assay for the rapid screening of irritation potential of surfactants, Molecular Toxicology 1, 525-536 1987)

### Beispiel 15

Hämolyse-Test,. H50 Wert nach 10 min., für Laurylethersulfat, APG (Plantaren 2000UP, Fa. Cognis) und **Lac-EG-C12.**
**Siehe** **Abbildung 6****.**

### Beispiel 16

Denatuierung, DI (in %), für Laurylethersulfat, APG (Plantaren 2000UP, Fa. Cognis) und **Lac-EG-12.**
**Siehe** **Abbildung 7****.**

### Beispiel 17

Quotient UD aus H50 und DI, für Laurylethersulfat, APG und **Lac-EG-C12.**
**Siehe** **Abbildung 8****.**

### Beispiele für Tensidmischungen

### Herstellung der Mischung in Beispiel 18:

Das Tensid wird in Wasser suspendiert und bis zur Einstellung einer klaren Lösung erhitzt, jedoch nicht über 60 °C. Anschließend wird die 50 °C warme Lösung mit Zitronensäure auf pH 5,5 eingestellt. Beim Abkühlen bildet sich ein Gel.

### Beispiel 18

### Gesichtsreinigungsgel

| | Gew.-% |
|---|---|
| Lac-EG-C12 | 3,00 |
| Zitronensäure | ad pH 5,5 |
| Wasser | ad 100,000 |

### Herstellung der Mischung in Beispiel 19:

Zu einer Lösung von Laurylethersulfat in Wasser wird das erfindungsgemäße Zuckertensid zugegeben und die Mischung bis zur Einstellung einer klaren Lösung erhitzt, jedoch nicht über 60 °C. Anschließend wird die 50 °C warme Lösung mit Zitronensäure auf pH 5,5 eingestellt.

### Beispiel 19

### Duschzubereitung

| | Gew.-% |
|---|---|
| Natrium Laureth Sulfat | 13,00 |
| Lac-EG-C12 | 2,00 |
| Zitronensäure | ad pH 5,5 |
| Wasser | ad 100,000 |

### Herstellung der Mischungen in Beispiel 20 und 21:

Zu einer Lösung von Laurylethersulfat in Wasser wird das erfindungsgemäße Zuckertensid zugegeben und die Mischung bis zur Einstellung einer klaren Lösung erhitzt, jedoch nicht über 60 °C. Anschließend wird die 50 °C warme Lösung mit Zitronensäure auf pH 5,5 eingestellt. Beim Abkühlen bildet sich ein Gel.

### Beispiel 20

### Rasiergel

| | Gew.-% |
|---|---|
| Natrium Laureth Sulfat | 11,00 |
| Lac-EG-C12 | 4,00 |
| Zitronensäure | ad pH 5,5 |
| Wasser | ad 100,000 |

### Beispiel 21

### Haushaltsreiniger

| | Gew.-% |
|---|---|
| Natrium Laureth Sulfat | 9,00 |
| Lac-EG-C12 | 6,00 |
| Zitronensäure | ad pH 5,5 |
| Wasser | ad 100,000 |

### Herstellung der Mischungen in Beispiel 22-30:

Zu einer Lösung des Cotensides in Wasser wird das erfindungsgemäße Zuckertensid zugegeben und die Mischung bis zur Einstellung einer klaren Lösung erhitzt, jedoch nicht über 60 °C. Anschließend wird die 50 °C warme Lösung mit Zitronensäure auf pH 5,5 eingestellt. Beim Abkühlen bildet sich ein Gel.

### Beispiel 22

### Make-up-Entferner

| | Gew.-% |
|---|---|
| Lac-EG-C12 | 2,50 |
| Natrium Cocoamphoacetat | 0,50 |
| Zitronensäure | ad pH 5,5 |
| Wasser | ad 100,000 |

### Beispiel 23

### Handwasch-Zubereitung

| | Gew.-% |
|---|---|
| Lac-EG-C12 | 2,00 |
| Natrium Cocoamphoacetat | 1,00 |
| Zitronensäure | ad pH 5,5 |
| Wasser | ad 100,000 |

### Beispiel 24

### Zahnpflege-Zubereitung

| | Gew.-% |
|---|---|
| Lac-EG-C12 | 1,50 |
| Natrium Cocoamphoacetat | 1,50 |
| Zitronensäure | ad pH 5,5 |
| Wasser | ad 100,000 |

### Beispiel 25

| | Gew.-% |
|---|---|
| Lac-EG-C12 | 2,50 |
| Cocamidopropyl Betain | 0,50 |
| Zitronensäure | ad pH 5,5 |
| Wasser | ad 100,000 |

### Beispiel 26

| | Gew.-% |
|---|---|
| Lac-EG-C12 | 2,00 |
| Cocamidopropyl Betain | 1,00 |
| Zitronensäure | ad pH 5,5 |
| Wasser | ad 100,000 |

### Beispiel 27

| | Gew.-% |
|---|---|
| Lac-EG-C12 | 1,50 |
| Cocamidopropyl Betain | 1,50 |
| Zitronensäure | ad pH 5,5 |
| Wasser | ad 100,000 |

### Beispiel 28

| | Gew.-% |
|---|---|
| Lac-EG-C12 | 2,50 |
| Dinatrium Cocoylglutamat | 0,50 |
| Zitronensäure | ad pH 5,5 |
| Wasser | ad 100,000 |

### Beispiel 29

| | Gew.-% |
|---|---|
| Lac-EG-C12 | 2,00 |
| Dinatrium Cocoylglutamat | 1,00 |
| Zitronensäure | ad pH 5,5 |
| Wasser | ad 100,000 |

### Beispiel 30

| | Gew.-% |
|---|---|
| Lac-EG-C12 | 1,50 |
| Dinatrium Cocoylglutamat | 1,50 |
| Zitronensäure | ad pH 5,5 |
| Wasser | ad 100,000 |

### Herstellung der Mischung in Beispiel 31:

Die erfindungsgemäße Tensidmischung wird bei Raumtemperatur in Wasser gelöst und mit Zitronensäure auf pH 5,5 eingestellt.

### Beispiel 31

### Tüchertränkmedium

| | Gew.-% |
|---|---|
| Lac-Laureth-2 | 3,00 |
| Zitronensäure | ad pH 5,5 |
| Wasser | ad 100,000 |

### Herstellung der Mischungen in Beispiel 32-34:

Die erfindungsgemäße Tensidmischung wird bei Raumtemperatur in einer Lösung von Laurylethersulfat gelöst und mit Zitronensäure auf pH 5,5 eingestellt.

### Beispiel 32

### Maschinengeschirrspülmittel

| | Gew.-% |
|---|---|
| Natrium Laureth Sulfat | 13,00 |
| Lac-Laureth-2 | 2,00 |
| Zitronensäure | ad pH 5,5 |
| Wasser | ad 100,000 |

### Beispiel 33

### Haarwaschmittel

| | Gew.-% |
|---|---|
| Natrium Laureth Sulfat | 11,00 |
| Lac-Laureth-2 | 4,00 |
| Zitronensäure | ad pH 5,5 |
| Wasser | ad 100,000 |

### Beispiel 34

### Fensterreiniger

| | Gew.-% |
|---|---|
| Natrium Laureth Sulfat | 9,00 |
| Lac-Laureth-2 | 6,00 |
| Zitronensäure | ad pH 5,5 |
| Wasser | ad 100,000 |

### Herstellung der Mischungen in Beispiel 35-43:

Die erfindungsgemäße Tensidmischung wird bei Raumtemperatur in einer Lösung des Cotensids gelöst und mit Zitronensäure auf pH 5,5 eingestellt.

### Beispiel 35

| | Gew.-% |
|---|---|
| Lac-Laureth-2 | 2,50 |
| Natrium Cocoamphoacetat | 0,50 |
| Zitronensäure | ad pH 5,5 |
| Wasser | ad 100,000 |

### Beispiel 36

| | Gew.-% |
|---|---|
| Lac-Laureth-2 | 2,00 |
| Natrium Cocoamphoacetat | 1,00 |
| Zitronensäure | ad pH 5,5 |
| Wasser | ad 100,000 |

### Beispiel 37

| | Gew.-% |
|---|---|
| Lac-Laureth-2 | 1,50 |
| Natrium Cocoamphoacetat | 1,50 |
| Zitronensäure | ad pH 5,5 |
| Wasser | ad 100,000 |

### Beispiel 38

| | Gew.-% |
|---|---|
| Lac-Laureth-2 | 2,50 |
| Cocamidopropyl Betain | 0,50 |
| Zitronensäure | ad pH 5,5 |
| Wasser | ad 100,000 |

### Beispiel 39

| | Gew.-% |
|---|---|
| Lac-Laureth-2 | 2,00 |
| Cocamidopropyl Betain | 1,00 |
| Zitronensäure | ad pH 5,5 |
| Wasser | ad 100,000 |

### Beispiel 40

| | Gew.-% |
|---|---|
| Lac-Laureth-2 | 1,50 |
| Cocamidopropyl Betain | 1,50 |
| Zitronensäure | ad pH 5,5 |
| Wasser | ad 100,000 |

### Beispiel 41

| | Gew.-% |
|---|---|
| Lac-Laureth-2 | 2,50 |
| Dinatrium Cocoylglutamat | 0,50 |
| Zitronensäure | ad pH 5,5 |
| Wasser | ad 100,000 |

### Beispiel 42

| | Gew.-% |
|---|---|
| Lac-Laureth-2 | 2,00 |
| Dinatrium Cocoylglutamat | 1,00 |
| Zitronensäure | ad pH 5,5 |
| Wasser | ad 100,000 |

### Beispiel 43

| | Gew.-% |
|---|---|
| Lac-Laureth-2 | 1,50 |
| Dinatrium Cocoylglutamat | 1,50 |
| Zitronensäure | ad pH 5,5 |
| Wasser | ad 100,000 |

### Herstellung der Mischungen in Beispiel 44 und 45:

Zu einer Lösung des erfindungsgemäßen Cotensids **Mal-EHG** wird das erfindungsgemäße Tensid **Lac-PG-C12** hinzugegeben und bis zur Einstellung einer klaren Lösung erhitzt, jedoch nicht über 60 °C. Die 50 °C warme Lösung wird mit Zitronensäure auf pH 5,5 eingestellt. Beim Abkühlen bildet sich ein Gel.

### Beispiel 44

| | Gew.-% |
|---|---|
| Lac-PG-C12 | 0,5 |
| Mal-EHG | 0,5 |
| Zitronensäure | ad pH 5,5 |
| Wasser | ad 100,000 |

### Beispiel 45

| | Gew.-% |
|---|---|
| Lac-PG-C12 | 1,5 |
| Mal-EHG | 1,5 |
| Zitronensäure | ad pH 5,5 |
| Wasser | ad 100,000 |

### Herstellung der Mischung in Beispiel 46:

Die erfindungsgemäßen Tenside werden in Wasser suspendiert und bis zur Einstellung einer klaren Lösung erhitzt, jedoch nicht über 60 °C. Die Lösung wird mit Zitronensäure auf pH 5,5 eingestellt.

### Beispiel 46

| | Gew.-% |
|---|---|
| Lac-α-EG-C12 | 2,4 |
| Lac-EG-C12 | 0,6 |
| Zitronensäure | ad pH 5,5 |
| Wasser | ad 100,000 |

### Herstellung der Mischung in Beispiel 47:

Das erfindungsgemäße Tensid wird bei Raumtemperatur in Wasser gelöst und mit Zitronensäure auf pH 5,5 eingestellt.

### Beispiel 47

| | Gew.-% |
|---|---|
| Lac-α-EG-C12 | 3,00 |
| Zitronensäure | ad pH 5,5 |
| Wasser | ad 100,000 |

### Herstellung der Mischungen in Beispiel 48-50:

Das erfindungsgemäße Tensid wird in einer Lösung des Cotensids in Wasser gelöst und mit Zitronensäure auf pH 5,5 eingestellt.

### Beispiel 48

| | Gew.-% |
|---|---|
| Lac-α-EG-C12 | 2,00 |
| Natrium Cocoamphoacetat | 1,00 |
| Zitronensäure | ad pH 5,5 |
| Wasser | ad 100,000 |

### Beispiel 49

| | Gew.-% |
|---|---|
| Lac-α-EG-C12 | 2,00 |
| Cocamidopropyl Betain | 1,00 |
| Zitronensäure | ad pH 5,5 |
| Wasser | ad 100,000 |

### Beispiel 50

| | Gew.-% |
|---|---|
| Lac-α-EG-C12 | 2,00 |
| Dinatrium Cocoylglutamat | 1,00 |
| Zitronensäure | ad pH 5,5 |
| Wasser | ad 100,000 |

## Patentansprüche

1. Kosmetische Zubereitung enthaltend eine Verbindung gemäß der Formel
A-(B)ₓ-C
wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 8 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat.

2. Zubereitung nach Patentanspruch 1 **dadurch gekennzeichnet, dass** das reduzierende Di- oder Trisaccharid A gewählt wird aus der Gruppe Maltose, Lactose, Melibiose, Cellobiose.

3. Zubereitung nach einem der vorangehenden Patentansprüche **dadurch gekennzeichnet, dass** ist und B gewählt wird aus der Gruppe Ethylenglycol, Diethylenglycol, Triethylenglycol, Tetraethylenglycol, Propan-1,2-diol, Propan-1,3-diol, Dipropylenglycol, Glycerin, Butandiol, Pentaerythrit.

4. Zubereitung nach einem der vorangehenden Patentansprüche **dadurch gekennzeichnet, dass** C ein linearer Fettalkoholrest ist.

5. Zubereitung nach einem der vorangehenden Patentansprüche **dadurch gekennzeichnet, dass** C ein gesättigter Fettalkoholrest ist.

6. Zubereitung nach einem der vorangehenden Patentansprüche enthaltend zusätzlich eine Verbindung gewählt unter
- Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
- Alkylsulfate, beispielsweise Natrium-, Ammonium- und TEA-Laurylsulfat,
- Natriumalkylsulfate wie Natriumcetearylsulfat

7. Zubereitung nach einem der vorangehenden Patentansprüche **dadurch gekennzeichnet, dass** sie schaumförmig oder schäumbar ist.

8. Verwendung einer Verbindung gemäß der Formel A-(B)ₓ-C
wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 8 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat
zur Verbesserung der Schäumbarkeit einer wasserhaltigen Zubereitung.

9. Verwendung von Produkt 14 gemäß Beispiel 7a, ((2'-Dodecyloxy-ethyl) 4-O-(β-D-galactopyranosyl)-β-D-glucopyranosid) und/oder ((2'-Dodecyloxy-propyl) 4-O-(β-D-galactopyranosyl)-β-D-glucopyranosid) als Gelbildner und/oder als Schaumbildner für wässrige Systeme.

10. Verfahren zur Herstellung einer Verbindung gemäß der Formel
A-(B)ₓ-C
wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 8 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat
durch Umsetzung eines β-Peracetats der Komponente A mit einer Lewis Säure und dem Polyolether Aₓ-B.

11. Verfahren zur Herstellung einer gemäß der Formel
A-(B)ₓ-C
wobei
A ein reduzierendes Di- oder Trisaccharid,
B ein mehrwertiger Alkohol mit 2 bis 4 C-Atomen oder dessen Derivat,
C ein verzweigter oder unverzweigter, gesättigter oder ungesättigter Fettalkoholrest mit 8 bis 22, besonders bevorzugt 8 bis 14 oder 14 bis 22, ganz besonders bevorzugt bis 20 C-Atomen ist,
A in 1-Stellung glycosidisch mit B verknüpft,
B mit C verethert ist und
x den Wert 1 bis 50 hat Verbindung nach einem der vorangehenden Patentansprüche durch Umglycosylierung aus Butylglycosiden, Propylglycosiden, Ethylglycosiden, Methylglycosiden mit Polyolethern Aₓ-B.
